# EUROPEAN PATENT APPLICATION

(11) **EP 3 896 085 A1**
(43) Date of publication of application: **20.10.2021**
(21) Application number: 19896274.8
(22) Date of filing: 11.12.2019
(51) Int. Cl.: C07K 16/24, C12N 15/13, C12N 15/63, C12N 5/10, C07K 19/00, A61K 39/395, A61P 11/00

(54) **ANTI-HUMAN INTERLEUKIN 5(IL-5) MONOCLONAL ANTIBODY AND USE THEREOF**

(30) Priority: 12.12.2018 CN 201811519048
(71) Applicant: Shanghai Pharmaexplorer Co., Ltd., Shanghai 201210 (CN)
(72) Inventor: YANG, Yizhen, Shanghai 201210 (CN); YANG, Songlin, Shanghai 201210 (CN); WU, Jian, Shanghai 201210 (CN); YANG, Xinxiu, Shanghai 201210 (CN); SHAO, Xiaohui, Shanghai 201210 (CN); ZHONG, Qin, Shanghai 201210 (CN); HU, Shaoping, Shanghai 201210 (CN); DUAN, Qing, Shanghai 201210 (CN); LIU, Lile, Shanghai 201210 (CN); WANG, Dongxu, Shanghai 201210 (CN); DAI, Chaohui, Shanghai 201210 (CN); WANG, Mengying, Shanghai 201210 (CN)
(74) Representative: Rondano, Davide
(86) International application number: PCT/CN2019/124644
(87) International publication number: WO 2020/119728

(57) **Abstract**

Disclosed in the present invention are an antibody targeting IL-5, a preparation method therefor and use thereof. In particular, disclosed in the present invention is a novel murine-derived or chimeric monoclonal antibody targeting IL-5. Also disclosed in the present invention is a method for preparing said monoclonal antibody. The monoclonal antibody of the present invention is capable of binding IL-5 antigen with high specificity, has high affinity and can well alleviate a series of asthma symptoms caused by IL-5, thereby achieving the effect of treating asthma.

Sequence listing

<110>  SHANGHAI PHARMAEXPLORER CO., LTD.

<120>  Anti-human interleukin-5 (IL-5) monoclonal antibody and use thereof

<130>  P2019-1835

<150>  CN201811519048.8
<151>  2018-12-12

<160>  50

<170>  PatentIn version 3.5

<210>  1
<211>  118
<212>  PRT
<213>  Artificial sequence

<220>
<223>  Heavy chain variable region

<400>  1

Glu Val Gln Leu Gln Gln Ser Gly Ala Glu Leu Val Lys Pro Gly Ala
1               5                   10                  15

Ser Val Lys Leu Ser Cys Thr Ala Ser Gly Phe Asn Ile Lys Asp Tyr
            20                  25                  30

Phe Ile His Trp Val Lys Gln Arg Thr Glu Gln Gly Leu Glu Trp Ile
        35                  40                  45

Gly Arg Ile Asp Thr Glu Asp Gly Glu Thr Lys Tyr Ala Pro Lys Phe
    50                  55                  60

Gln Gly Lys Ala Thr Ile Thr Ile Asp Thr Ser Ser Asn Thr Ala Tyr
65                  70                  75                  80

Leu Tyr Phe Ser Ser Leu Thr Ser Glu Asp Thr Ala Met Tyr Tyr Cys
                85                  90                  95

Ala Arg Tyr Gly Asn Tyr Val Ala Met Asp Tyr Trp Gly Gln Gly Thr
            100                 105                 110

Ser Val Thr Val Ser Ser
        115

<210>  2
<211>  354
<212>  DNA
<213>  Artificial sequence

<220>

<223>  Heavy chain variable region

<400>  2
gaggttcagc tgcagcagtc tggggcagag cttgtgaaac caggggcctc agtcaagttg      60

tcctgcacag cttctggctt caacattaaa gactacttta tacactgggt gaagcagagg     120

actgaacagg gcctggagtg gattggaagg attgatactg aggatggtga aactaaatat     180

gccccgaaat tccagggcaa ggccactata acaatagaca catcctccaa tacagcctac     240

ctgtatttca gcagcctgac atctgaggac actgccatgt attactgtgc tagatatggt     300

aattacgtcg ctatggacta ctggggtcaa ggaacctcag tcaccgtctc ctca           354


<210>  3
<211>  5
<212>  PRT
<213>  Artificial sequence

<220>
<223>  Heavy chain CDR1

<400>  3

Asp Tyr Phe Ile His
1               5


<210>  4
<211>  17
<212>  PRT
<213>  Artificial sequence

<220>
<223>  Heavy chain CDR2

<400>  4

Arg Ile Asp Thr Glu Asp Gly Glu Thr Lys Tyr Ala Pro Lys Phe Gln
1               5                   10                  15


Gly




<210>  5
<211>  9
<212>  PRT
<213>  Artificial sequence

<220>
<223>  Heavy chain CDR3

<400>  5

Tyr Gly Asn Tyr Val Ala Met Asp Tyr
1               5


<210>  6

<211>  106
<212>  PRT
<213>  Artificial sequence

<220>
<223>  Light chain variable region

<400>  6

Gln Ile Val Leu Thr Gln Ser Pro Ala Leu Met Ser Ala Ser Pro Gly
1               5                   10                  15

Glu Lys Val Thr Met Thr Cys Ser Ala Ser Ser Ser Val Ser Tyr Met
            20                  25                  30

Phe Trp Tyr Gln Gln Lys Pro Ser Ser Ser Pro Lys Pro Trp Ile Tyr
        35                  40                  45

Leu Thr Ser Lys Leu Ala Ser Gly Val Pro Ala Arg Phe Ser Gly Ser
    50                  55                  60

Gly Ser Gly Thr Ser Tyr Ser Leu Thr Ile Ser Ser Val Glu Ala Glu
65                  70                  75                  80

Asp Ala Ala Thr Tyr Tyr Cys Gln Gln Trp Asn Ser Asn Pro Tyr Thr
                85                  90                  95

Phe Gly Gly Gly Thr Lys Leu Glu Ile Lys
            100                 105

<210>  7
<211>  318
<212>  DNA
<213>  Artificial sequence

<220>
<223>  Light chain variable region

<400>  7
caaattgttc tcacccagtc tccagcactc atgtctgcat ctccagggga gaaggtcacc      60

atgacctgca gtgccagctc aagtgtaagt tacatgttct ggtaccagca gaagccaagt     120

tcctccccca aaccctggat ttatctcaca tccaagctgg cttctggagt ccctgctcgc     180

ttcagtggca gtgggtctgg gacctcttac tctctcacaa tcagcagcgt ggaggctgaa     240

gatgctgcca cttattactg tcagcagtgg aacagtaacc cgtacacgtt cggagggggg     300

accaagctgg aaataaaa                                                   318

<210>  8
<211>  10
<212>  PRT
<213>  Artificial sequence

<220>
<223>  Light chain CDR1

<400>  8

Ser Ala Ser Ser Ser Val Ser Tyr Met Phe
1               5                   10


<210>  9
<211>  7
<212>  PRT
<213>  Artificial sequence

<220>
<223>  Light chain CDR2

<400>  9

Leu Thr Ser Lys Leu Ala Ser
1               5


<210>  10
<211>  9
<212>  PRT
<213>  Artificial sequence

<220>
<223>  Light chain CDR3

<400>  10

Gln Gln Trp Asn Ser Asn Pro Tyr Thr
1               5


<210>  11
<211>  115
<212>  PRT
<213>  Artificial sequence

<220>
<223>  Heavy chain variable region

<400>  11

Gln Val Gln Leu Arg Gln Ser Gly Pro Gly Leu Val Gln Pro Ser Gln
1               5                   10                  15


Ser Leu Ser Ile Thr Cys Thr Val Ser Gly Phe Ser Leu Thr Ser Tyr
            20                  25                  30


Gly Val His Trp Val Arg Gln Ser Pro Gly Lys Gly Leu Glu Trp Leu
        35                  40                  45


Gly Val Ile Trp Ser Gly Gly Ser Thr Asp Tyr Asn Ala Val Phe Met
    50                  55                  60

Ser Arg Leu Ser Ile Ser Lys Asp Ser Ser Lys Ser Gln Val Phe Phe
65                  70                  75                  80

Lys Met Asn Ser Leu Gln Thr Asp Asp Thr Ala Ile Tyr Tyr Cys Ala
                85                  90                  95

Arg Asn Arg Tyr Val Leu Asp Tyr Trp Gly Gln Gly Thr Ser Val Thr
            100                 105                 110

Val Ser Ser
        115

<210>  12
<211>  345
<212>  DNA
<213>  Artificial sequence

<220>
<223>  Heavy chain variable region

<400>  12
caggtgcagc tgaggcagtc aggacctggc ctggtgcagc cctcacagag cctgtccatc      60

acctgcacag tctctggttt ctcattaact agctatggtg tacactgggt tcgccagtct     120

ccaggaaagg gtctggagtg gctgggagtg atatggagtg gtggaagcac agactataat     180

gcagttttca tgtccagact gagcatcagc aaggacagtt ccaagagcca agttttcttt     240

aaaatgaaca gtctgcaaac tgatgacaca gccatatatt actgtgccag aaaccgttac     300

gttttggact actggggtca aggaacctca gtcaccgtct cctca                     345

<210>  13
<211>  5
<212>  PRT
<213>  Artificial sequence

<220>
<223>  Heavy chain CDR1

<400>  13

Ser Tyr Gly Val His
1               5

<210>  14
<211>  16
<212>  PRT
<213>  Artificial sequence

<220>
<223>  Heavy chain CDR2

<400>  14

Val Ile Trp Ser Gly Gly Ser Thr Asp Tyr Asn Ala Val Phe Met Ser
1               5                   10                  15

<210>  15
<211>  7
<212>  PRT
<213>  Artificial sequence

<220>
<223>  Heavy chain CDR3

<400>  15

Asn Arg Tyr Val Leu Asp Tyr
1               5

<210>  16
<211>  113
<212>  PRT
<213>  Artificial sequence

<220>
<223>  Light chain variable region

<400>  16

Asp Ile Val Met Thr Gln Ser Pro Ser Ser Leu Ser Val Ser Ala Gly
1               5                   10                  15

Glu Arg Val Thr Met Arg Cys Lys Ser Ser Gln Ser Leu Leu Thr Ser
            20                  25                  30

Gly Asp Gln Lys Asn His Leu Ala Trp Tyr Gln Gln Lys Pro Gly Gln
        35                  40                  45

Pro Pro Lys Leu Leu Ile Tyr Gly Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Thr Ser Val Gln Ala Glu Asp Leu Ala Val Tyr Tyr Cys Gln Asn
                85                  90                  95

Asp His Ser Phe Pro Leu Thr Phe Gly Ala Gly Thr Lys Leu Glu Leu
            100                 105                 110

Lys

<210>  17
<211>  339
<212>  DNA

<213>  Artificial sequence

<220>
<223>  Light chain variable region

<400>  17
gacattgtga tgacacagtc gccatcctcc ctgagtgtgt cagcaggaga gagggtcact      60

atgaggtgca agtccagtca gagtctgtta accagtggag atcaaaagaa ccacttggcc     120

tggtaccagc agaaaccagg gcagcctcct aaactgttga tctacggggc atccactagg     180

gaatctgggg tccctgatcg cttcacaggc agtggatctg gaaccgattt cactcttacc     240

atcaccagtg tgcaggctga agacctggca gtttattact gtcagaatga tcatagtttt     300

ccgctcacgt tcggtgctgg gaccaagctg gagctgaaa                            339


<210>  18
<211>  17
<212>  PRT
<213>  Artificial sequence

<220>
<223>  Light chain CDR1

<400>  18

Lys Ser Ser Gln Ser Leu Leu Thr Ser Gly Asp Gln Lys Asn His Leu
1               5                   10                  15


Ala




<210>  19
<211>  7
<212>  PRT
<213>  Artificial sequence

<220>
<223>  Light chain CDR2

<400>  19

Gly Ala Ser Thr Arg Glu Ser
1               5


<210>  20
<211>  9
<212>  PRT
<213>  Artificial sequence

<220>
<223>  Light chain CDR3

<400>  20

Gln Asn Asp His Ser Phe Pro Leu Thr
1               5

<210>  21
<211>  115
<212>  PRT
<213>  Artificial sequence

<220>
<223>  Heavy chain variable region

<400>  21

Gln Val Gln Leu Lys Gln Ser Gly Pro Gly Leu Val Gln Pro Ser Gln
1               5                   10                  15

Ser Leu Ser Ile Thr Cys Thr Val Ser Gly Phe Ser Leu Ser Asn Tyr
            20                  25                  30

Gly Val His Trp Val Arg Gln Ser Pro Gly Lys Gly Leu Glu Trp Leu
        35                  40                  45

Gly Val Ile Trp Ser Gly Gly Ser Thr Asp Tyr Asn Ala Ala Phe Lys
    50                  55                  60

Ser Arg Leu Ser Ile Thr Lys Asp Asn Ser Lys Ser Gln Val Val Phe
65                  70                  75                  80

Arg Met Asn Ser Leu Gln Thr Asp Asp Thr Ala Thr Tyr Tyr Cys Ala
                85                  90                  95

Arg Asn Lys Asp Phe Leu Asp Tyr Trp Gly Gln Gly Thr Lys Phe Thr
            100                 105                 110

Val Ser Ser
        115

<210>  22
<211>  345
<212>  DNA
<213>  Artificial sequence

<220>
<223>  Heavy chain variable region

<400>  22
caggtgcagc tgaagcagtc aggacctggc ctagtgcagc cctcacagag cctgtccatc      60

acctgcacag tctctggttt ctcattaagt aactatggtg tacactgggt tcgccagtct     120

ccaggaaagg gtctggagtg gctgggagtg atatggagtg gtggaagcac agactataat     180

gcagctttca aatccagact gagcatcacc aaggacaatt ccaagagcca agttgtcttt     240

agaatgaaca gtctgcaaac tgatgacaca gccacttatt actgtgccag aaataaggac     300

ttcttggact actggggcca aggcaccaag ttcacagtct cctca                    345

<210>  23
<211>  5
<212>  PRT
<213>  Artificial sequence

<220>
<223>  Heavy chain CDR1

<400>  23

Asn Tyr Gly Val His
1               5

<210>  24
<211>  16
<212>  PRT
<213>  Artificial sequence

<220>
<223>  Heavy chain CDR2

<400>  24

Val Ile Trp Ser Gly Gly Ser Thr Asp Tyr Asn Ala Ala Phe Lys Ser
1               5                   10                  15

<210>  25
<211>  7
<212>  PRT
<213>  Artificial sequence

<220>
<223>  Heavy chain CDR3

<400>  25

Asn Lys Asp Phe Leu Asp Tyr
1               5

<210>  26
<211>  113
<212>  PRT
<213>  Artificial sequence

<220>
<223>  Light chain variable region

<400>  26

Asp Ile Val Met Thr Gln Ser Pro Ser Ser Leu Thr Val Thr Ala Gly
1               5                   10                  15

Glu Lys Val Thr Met Thr Cys Lys Ser Ser Gln Gly Leu Leu Asn Ser
            20                  25                  30

Gly Asn Gln Lys Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Gln Asn
                85                  90                  95

Asp Tyr His Phe Pro Leu Thr Phe Gly Ala Gly Thr Lys Leu Glu Leu
            100                 105                 110

Lys

<210>  27
<211>  339
<212>  DNA
<213>  Artificial sequence

<220>
<223>  Light chain variable region

<400>  27
gacattgtga tgacacagtc tccatcctcc ctgactgtga cagcaggaga gaaggtcact      60

atgacctgca agtccagtca gggtctgtta aacagtggaa atcaaaagaa ctacttgacc     120

tggtaccagc agaaaccagg gcagcctcct aaactgttga tctactgggc atccaccagg     180

gaatctgggg tccctgatcg cttcacaggc agtggatctg gaacagattt cactctcacc     240

atcagcagtg tgcaggctga agacctggca gtttattact gtcagaatga ttatcatttt     300

ccgctcacgt tcggagctgg gaccaagctg gaactgaaa                            339

<210>  28
<211>  17
<212>  PRT
<213>  Artificial sequence

<220>
<223>  Light chain CDR1

<400>  28

Lys Ser Ser Gln Gly Leu Leu Asn Ser Gly Asn Gln Lys Asn Tyr Leu
1               5                   10                  15

Thr

<210>  29
<211>  7
<212>  PRT
<213>  Artificial sequence

<220>
<223>  Light chain CDR2

<400>  29

Trp Ala Ser Thr Arg Glu Ser
1               5


<210>  30
<211>  9
<212>  PRT
<213>  Artificial sequence

<220>
<223>  Light chain CDR3

<400>  30

Gln Asn Asp Tyr His Phe Pro Leu Thr
1               5


<210>  31
<211>  115
<212>  PRT
<213>  Artificial sequence

<220>
<223>  Heavy chain variable region

<400>  31

Gln Val Gln Leu Gln Gln Pro Gly Ala Glu Leu Val Lys Pro Gly Ala
1               5                   10                  15

Ser Val Lys Leu Ser Cys Lys Ala Ser Gly Tyr Thr Phe Thr Ser Tyr
            20                  25                  30

Trp Ile Thr Trp Val Lys Gln Arg Pro Gly Gln Gly Leu Glu Trp Ile
        35                  40                  45

Gly Asp Ile Tyr Pro Gly Ser Gly Ser Thr Phe Tyr Asn Glu Lys Phe
    50                  55                  60

Lys Ser Glu Ala Thr Leu Thr Val Asp Thr Ser Ser Ser Thr Ala Tyr
65                  70                  75                  80

Met Gln Leu Ser Ser Leu Thr Ser Glu Asp Ser Ala Val Phe Tyr Cys
                85                  90                  95

Ser Thr Glu Thr Thr Leu Gly Tyr Trp Gly Gln Gly Thr Thr Leu Thr
            100                 105                 110

Val Ser Ser
        115

<210>  32
<211>  345
<212>  DNA
<213>  Artificial sequence

<220>
<223>  Heavy chain variable region

<400>  32
caggtccaac tgcagcagcc tggggctgag cttgtgaagc ctggggcttc agtgaagctg      60

tcctgcaagg cttctggcta caccttcacc agctactgga taacctgggt gaagcagagg     120

cctggacaag gccttgagtg gattggagat atttatcctg gtagtggtag tactttctac     180

aatgagaagt tcaagagcga ggccacactg actgtagaca catcctccag cacagcctac     240

atgcagctca gcagcctgac atctgaggac tctgcggtct tttactgttc aaccgagact     300

accctgggct actggggcca aggcaccact ctcacagtct cctca                     345

<210>  33
<211>  5
<212>  PRT
<213>  Artificial sequence

<220>
<223>  Heavy chain CDR1

<400>  33

Ser Tyr Trp Ile Thr
1               5

<210>  34
<211>  17
<212>  PRT
<213>  Artificial sequence

<220>
<223>  Heavy chain CDR2

<400>  34

Asp Ile Tyr Pro Gly Ser Gly Ser Thr Phe Tyr Asn Glu Lys Phe Lys
1               5                   10                  15

Ser

<210>  35

<211>  6
<212>  PRT
<213>  Artificial sequence

<220>
<223>  Heavy chain CDR3

<400>  35

Glu Thr Thr Leu Gly Tyr
1               5


<210>  36
<211>  112
<212>  PRT
<213>  Artificial sequence

<220>
<223>  Light chain variable region

<400>  36

Asp Val Leu Met Thr Gln Thr Pro Leu Ser Leu Pro Val Ser Leu Gly
1               5                   10                  15


Asp Gln Ala Ser Ile Ser Cys Arg Ser Ser Gln Ser Ile Val His Asn
            20                  25                  30


Asn Gly Asn Thr Tyr Leu Glu Trp Tyr Leu Gln Lys Pro Gly Gln Ser
        35                  40                  45


Pro Lys Leu Leu Ile Tyr Lys Val Ser Asn Arg Phe Ser Gly Val Pro
    50                  55                  60


Asp Arg Phe Ser Gly Ser Gly Ser Gly Thr Asp Phe Thr Leu Lys Ile
65                  70                  75                  80


Ser Arg Val Glu Ala Glu Asp Leu Gly Val Tyr Tyr Cys Phe Gln Gly
                85                  90                  95


Ser His Val Pro Phe Thr Phe Gly Ser Gly Thr Lys Leu Glu Ile Lys
            100                 105                 110


<210>  37
<211>  336
<212>  DNA
<213>  Artificial sequence

<220>
<223>  Light chain variable region

<400>  37
gatgttttga tgacccaaac tccactctcc ctgcctgtca gtcttggaga tcaagcctcc        60

atctcttgca gatctagtca gagcattgta cataataatg gaaacaccta tttagaatgg       120

tacctgcaga aaccaggcca gtctccaaag ctcctgatct acaaagtttc caaccgattt     180

tctggggtcc cagacaggtt cagtggcagt ggatcaggga cagatttcac actcaagatc     240

agcagagtgg aggctgagga tctgggagtt tattactgct ttcaaggttc acatgttcca     300

ttcacgttcg gctcggggac aaagttggaa ataaaa                               336

<210>  38
<211>  16
<212>  PRT
<213>  Artificial sequence

<220>
<223>  Light chain CDR1

<400>  38

Arg Ser Ser Gln Ser Ile Val His Asn Asn Gly Asn Thr Tyr Leu Glu
1               5                   10                  15

<210>  39
<211>  7
<212>  PRT
<213>  Artificial sequence

<220>
<223>  Light chain CDR2

<400>  39

Lys Val Ser Asn Arg Phe Ser
1               5

<210>  40
<211>  9
<212>  PRT
<213>  Artificial sequence

<220>
<223>  Light chain CDR3

<400>  40

Phe Gln Gly Ser His Val Pro Phe Thr
1               5

<210>  41
<211>  116
<212>  PRT
<213>  Artificial sequence

<220>
<223>  Heavy chain variable region

<400>  41

Glu Val His Leu Gln Gln Ser Gly Ala Glu Leu Val Lys Pro Gly Ala

1               5                   10                  15

Ser Val Lys Leu Ser Cys Thr Ala Ser Gly Phe Asn Ile Lys Asp Tyr
            20                  25                  30

Tyr Met His Trp Val Lys Gln Arg Thr Glu Gln Gly Leu Glu Trp Ile
        35                  40                  45

Gly Arg Ile Asp Pro Glu Asp Gly Glu Thr Lys Tyr Ala Pro Lys Phe
    50                  55                  60

Gln Gly Gln Ala Thr Ile Thr Ala Asp Thr Ser Ser Asn Thr Ala Asn
65                  70                  75                  80

Leu Gln Leu Ser Ser Leu Thr Ser Glu Asp Thr Ala Val Tyr Tyr Cys
                85                  90                  95

Ala Arg Tyr Gly Asn Phe Pro Asp Tyr Trp Gly Pro Gly Thr Thr Leu
            100                 105                 110

Thr Val Ser Ser
        115

<210>  42
<211>  348
<212>  DNA
<213>  Artificial sequence

<220>
<223>  Heavy chain variable region

<400>  42
gaggttcacc tgcagcagtc tggggcagag cttgtgaagc caggggcctc agtcaagttg      60

tcctgcacag cttctggctt caacattaaa gactactata tgcactgggt gaagcagagg     120

actgaacagg gcctggagtg gattggaagg attgatcctg aggatggtga aactaaatat     180

gccccgaaat tccagggcca ggccactata acagcggaca catcctccaa cactgccaac     240

ctgcagctca gcagcctgac atctgaggac actgccgtct attactgtgc gagatatggt     300

aacttccctg actactgggg cccaggcacc actctcacag tctcctca                  348

<210>  43
<211>  5
<212>  PRT
<213>  Artificial sequence

<220>
<223>  Heavy chain CDR1

<400>  43

Asp Tyr Tyr Met His
1               5

<210>  44
<211>  17
<212>  PRT
<213>  Artificial sequence

<220>
<223>  Heavy chain CDR2

<400>  44

Arg Ile Asp Pro Glu Asp Gly Glu Thr Lys Tyr Ala Pro Lys Phe Gln
1               5                   10                  15

Gly

<210>  45
<211>  7
<212>  PRT
<213>  Artificial sequence

<220>
<223>  Heavy chain CDR3

<400>  45

Tyr Gly Asn Phe Pro Asp Tyr
1               5

<210>  46
<211>  106
<212>  PRT
<213>  Artificial sequence

<220>
<223>  Light chain variable region

<400>  46

Gln Ile Val Leu Thr Gln Ser Pro Ala Leu Met Ser Ala Ser Pro Gly
1               5                   10                  15

Glu Lys Val Thr Met Thr Cys Ser Ala Ser Ser Ser Val Ser Tyr Met
            20                  25                  30

Tyr Trp Tyr Gln Gln Lys Pro Arg Ser Ser Pro Lys Pro Trp Ile Tyr
        35                  40                  45

Leu Thr Ser Lys Leu Ala Ser Gly Val Pro Ala Arg Phe Ser Gly Ser
    50                  55                  60

Gly Ser Gly Thr Ser Tyr Ser Leu Thr Ile Ser Ser Met Glu Ala Glu

65                  70                  75                  80

Asp Ala Ala Thr Tyr Tyr Cys Gln Gln Trp Asn Ser Asn Pro Tyr Thr
                85                  90                  95

Phe Gly Gly Gly Thr Lys Leu Glu Ile Lys
            100                 105

<210>  47
<211>  318
<212>  DNA
<213>  Artificial sequence

<220>
<223>  Light chain variable region

<400>  47
caaattgttc tcacccagtc tccagcactc atgtctgcat ctccagggga gaaggtcacc      60

atgacctgca gtgccagctc aagtgtaagt tacatgtact ggtaccagca gaagccaaga     120

tcctcaccca aaccctggat ttatctcaca tccaaactgg cttctggagt ccctgctcgc     180

ttcagtggca gtgggtctgg gacctcttac tctctcacaa tcagcagcat ggaggctgaa     240

gatgctgcca cttattactg ccagcagtgg aatagtaacc cgtacacgtt cggagggggg     300

accaagctgg aaataaaa                                                   318

<210>  48
<211>  10
<212>  PRT
<213>  Artificial sequence

<220>
<223>  Light chain CDR1

<400>  48

Ser Ala Ser Ser Ser Val Ser Tyr Met Tyr
1               5                   10

<210>  49
<211>  7
<212>  PRT
<213>  Artificial sequence

<220>
<223>  Light chain CDR2

<400>  49

Leu Thr Ser Lys Leu Ala Ser
1               5

<210>  50
<211>  9

<212>  PRT
<213>  Artificial sequence

<220>
<223>  Light chain CDR3

<400>  50

Gln Gln Trp Asn Ser Asn Pro Tyr Thr
1               5

## Description

### Technical field

The invention relates to the biomedical field, in particular to an IL-5 antibody as well as preparation method and application thereof.

### Background art

Bronchial asthma (asthma for short) is a common chronic airway inflammatory disease, which is usually accompanied by increased airway reactivity and recurrent symptoms such as wheezing, shortness of breath, chest tightness and/or cough. After 1970s, asthma became widespread. By 2011, about 235 million to 300 million people worldwide were affected, and about 250,000 people lost their lives as a result. Clinically, asthma is usually controlled by inhaling glucocorticoids such as steroids. For patients with unsatisfactory disease control, adjuvant treatment such as inhaled long-acting β 2 agonist, sustained-release theophylline or leukotriene regulator can be added. However, about 10% of patients still can't control their illness well through conventional treatment. These patients often need to give large doses of glucocorticoids orally or intravenously to control their illness, which is still accompanied by high death rate. Not only the quality of life of patients is greatly affected, but also the direct medical expenses and indirect costs will cause huge personal and social economic burdens.

In recent years, it has been reported that reversible airway obstruction leads to nonspecific bronchial hyperreactivity asthma, which mainly depends on the generation of a chronic inflammatory reaction at the bronchial mucosal level, and its typical feature is the infiltration of macrophages, lymphocytes and eosinophils. Eosinophils play an important role in initiating typical diseases of mucosal injury. Eosinophils are one of the normal leukocytes in human body, which have the function of killing bacteria and parasites, and are also extremely important cells in the process of immune reaction and allergic reaction. Eosinophils can release the contents of granules, causing tissue damage and promoting inflammation. In addition, there have been reports of increased eosinophils in bronchial secretions and lung parenchyma in the circulatory system of patients with chronic asthma. And the number of eosinophils in blood is used as an important index to judge the severity of lung function diseases.

With the in-depth study of asthma, eosinophils are considered to be the main pathogenesis of various inflammatory diseases, including allergic diseases related to allergic reaction of lung tissue. In most asthma patients, Th2 cells over-release soluble cytokines and induce IgE production, which leads to degranulation of mast cells and eosinophils, thus causing airway allergic reaction and chronic airway inflammation caused by multiple inflammatory cells. Among them, interleukin-5(hereinafter referred to as IL-5) plays a very important role in this process. IL-5 is a multivariate cytokine secreted by Th2 cells and mast cells, and is a disulfide-linked homodimer glycoprotein. Its molecular weight is about 24KDa, and its gene is located on chromosome 5, which is closely linked with other cytokines such as interleukin-3 (IL-3), interleukin-4 (IL-4) and granulocyte macrophage stimulating factor (GM-CSF). It has been proved that IL-5 acts specifically on eosinophils, mainly regulating their growth, differentiation, maturation, survival, activation and are released from bone marrow, blood and tissues.

In recent years, many animal experiments have been confirmed that IL-5 can increase the number of eosinophils in peripheral blood and tissues. After using antimouse IL-5 antibody, eosinophils decreased significantly. At the same time, high concentration of IL-5 was also found in bronchoalveolar lavage fluid of asthma patients.

Therefore, it is urgent to develop efficient IL-5 antibodies in the art to benefit more patients.

### Summary of the invention

In order to overcome the current lack of fully human IL-5 antibody and the shortcomings of existing IL-5 antibodies in terms of activity, the present invention provides an IL-5 antibody with high affinity and strong specificity, and a preparation method and application thereof.

In a first aspect of the present invention, it provides a heavy chain variable region of an antibody, wherein the heavy chain variable region comprises complementary determining regions or CDRs selected from the group consisting of:
VH-CDR1 shown in SEQ ID NO: 10n+3,
VH-CDR2 shown in SEQ ID NO: 10n+4, and
VH-CDR3 shown in SEQ ID NO: 10n+5;
   wherein each n is independently 0, 1, 2, 3, or 4;
   wherein any one of the above amino acid sequences further comprises a derivative sequence which is obtained through optional addition, deletion, modification and/or substitution of at least one amino acid and is capable of retaining the binding affinity to IL-5.

In another preferred embodiment, the heavy chain variable region has the amino acid sequence shown in SEQ ID NO: 10n+1, wherein n is 0, 1, 2, 3, or 4.

In another preferred embodiment, the heavy chain variable region has the amino acid sequence as shown in SEQ ID NO: 1.

In another preferred embodiment, the heavy chain variable region has the amino acid sequence as shown in SEQ ID NO: 11.

In another preferred embodiment, the heavy chain variable region has the amino acid sequence as shown in SEQ ID NO: 21.

In another preferred embodiment, the heavy chain variable region has the amino acid sequence as shown in SEQ ID NO: 31.

In another preferred embodiment, the heavy chain variable region has the amino acid sequence as shown in SEQ ID NO: 41.

In a second aspect of the present invention, it provides a heavy chain of an antibody, which has the heavy chain variable region of the first aspect of the present invention.

In another preferred embodiment, the heavy chain further comprises a heavy chain constant region.

In another preferred embodiment, the heavy chain constant region is of human or mouse.

In another preferred embodiment, the heavy chain constant region is a human antibody heavy chain IgG4 constant region.

In a third aspect of the present invention, it provides a light chain variable region of an antibody, wherein the light chain variable region comprises complementary determining regions or CDRs selected from the group consisting of:
VL-CDR1 shown in SEQ ID NO: 10n+8,
VL-CDR2 shown in SEQ ID NO: 10n+9, and
VL-CDR3 shown in SEQ ID NO: 10n+10;
   wherein each n is independently 0, 1, 2, 3, or 4;
   wherein any one of the above amino acid sequences further comprises a derivative sequence which is obtained through optional addition, deletion, modification and/or substitution of at least one amino acid and is capable of retaining the binding affinity to IL-5.

In another preferred embodiment, the light chain variable region has the amino acid sequence shown in SEQ ID NO: 10n+6, wherein n is 0, 1, 2, 3, or 4.

In another preferred embodiment, the light chain variable region has the amino acid sequence as shown in SEQ ID NO: 6.

In another preferred embodiment, the light chain variable region has the amino acid sequence as shown in SEQ ID NO: 16.

In another preferred embodiment, the light chain variable region has the amino acid sequence as shown in SEQ ID NO: 26.

In another preferred embodiment, the light chain variable region has the amino acid sequence as shown in SEQ ID NO: 36.

In another preferred embodiment, the light chain variable region has the amino acid sequence as shown in SEQ ID NO: 46.

In a fourth aspect of the present invention, it provides a light chain of an antibody, which has the light chain variable region of the third aspect of the present invention.

In another preferred embodiment, the light chain further comprises a light chain constant region.

In another preferred embodiment, the light chain constant region is of human or mouse.

In another preferred embodiment, the light chain constant region is a human antibody light chain kappa constant region.

In a fifth aspect of the present invention, it provides an antibody having:
(1) the heavy chain variable region of the first aspect of the present invention; and/or
(2) the light chain variable region of the third aspect of the present invention;
   alternatively, the antibody has: the heavy chain of the second aspect of the present invention; and/or the light chain of the fourth aspect of the present invention,
   wherein any one of the above amino acid sequences further comprises a derivative sequence which is obtained through optional addition, deletion, modification and/or substitution of at least one amino acid and is capable of retaining the binding affinity to IL-5.

In another preferred embodiment, the amino acid sequence of any of the above-mentioned CDRs comprises a derivative CDR sequence with 1, 2 or 3 amino acids added, deleted, modified and/or substituted, and the derivative antibody comprising the VH and VL containing the derivative CDR sequence can retain the binding affinity to IL-5.

In another preferred embodiment, the ratio (F1/F0) of the binding affinity F1 between the derivatized antibody and IL-5 to the binding affinity F0 between the corresponding non-derivatized antibody and IL-5 is 0.5-2, preferably 0.7-1.5, and more preferably 0.8-1.2.

In another preferred embodiment, the number of added, deleted, modified and/or substituted amino acids is 1-5 (such as 1-3, preferably 1-2, more preferably 1).

In another preferred embodiment, the derivative sequence with at least one amino acid added, deleted, modified, and/or substituted, which can retain the binding affinity to IL-5, is an amino acid sequence having a homology or sequence identity of at least 96%.

In another preferred embodiment, the antibody further comprises a heavy chain constant region and/or a light chain constant region.

In another preferred embodiment, the heavy chain constant region is of human, and/or the light chain constant region is of human.

In another preferred embodiment, the heavy chain constant region is a human antibody heavy chain IgG4 constant region, and the light chain constant region is a human antibody light chain kappa constant region.

In another preferred embodiment, the heavy chain variable region of the antibody further comprises human-derived framework region, and/or the light chain variable region of the antibody further comprises human-derived framework region.

In another preferred embodiment, the heavy chain variable region of the antibody further comprises murine-derived framework region, and/or the light chain variable region of the antibody further comprises murine-derivedframework region.

In another preferred embodiment, the antibody is selected from the group consisting of: animal-derived antibodies, chimeric antibodies, humanized antibodies, fully human antibodies, and a combination thereof.

In another preferred embodiment, the ratio (Z1/Z0) of the immunogenicity Z1 of the chimeric antibody in human to the immunogenicity Z0 of the non-chimeric antibody (such as murine-derived antibody) in human is 0-0.5, preferably 0-0.2, more preferably 0-0.05 (e.g. 0.001-0.05).

In another preferred embodiment, the antibody is a partially or fully humanized or fully human monoclonal antibody.

In another preferred embodiment, the antibody is a double-chain antibody or a single-chain antibody.

In another preferred embodiment, the antibody is a full-length antibody protein or an antigen-binding fragment.

In another preferred embodiment, the antibody is a bispecific antibody or a multispecific antibody.

In another preferred embodiment, the antibody is in the form of a drug conjugate.

In another preferred embodiment, the antibody has one or more properties selected from the group consisting of:
(a) inhibiting the occurrence and/or development of asthma;
(b) relieving asthma symptoms.

In another preferred embodiment, the antibody comprises the heavy chain variable region according to the first aspect of the present invention and the light chain variable region according to the third aspect of the present invention;
wherein, the heavy chain variable region and the light chain variable region comprise CDRs selected from the group consisting of:

| VH-CDR1 Sequence number | VH-CDR2 Sequence number | VH-CDR3 Sequence number | VL-CDR1 Sequence number | VL-CDR2 Sequence number | VL-CDR3 Sequence number |
|---|---|---|---|---|---|
| 3 | 4 | 5 | 8 | 9 | 10 |
| 13 | 14 | 15 | 18 | 19 | 20 |
| 23 | 24 | 25 | 28 | 29 | 30 |
| 33 | 34 | 35 | 38 | 39 | 40 |
| 43 | 44 | 45 | 48 | 49 | 50 |

wherein any one of the above amino acid sequences further comprises a derivative sequence which is obtained through optional addition, deletion, modification and/or substitution of at least one amino acid and is capable of retaining the binding affinity to IL-5.

In another preferred embodiment, the antibody comprises the heavy chain variable region according to the first aspect of the present invention and the light chain variable region according to the third aspect of the present invention; wherein,
the heavy chain variable region comprises the following three complementary determining regions or CDRs:
   VH-CDR1 shown in SEQ ID NO: 3,
   VH-CDR2 shown in SEQ ID NO: 4, and
   VH-CDR3 shown in SEQ ID NO: 5;
the light chain variable region comprises the following three complementary determining regions or CDRs:
   VL-CDR1 shown in SEQ ID NO: 8,
   VL-CDR2 shown in SEQ ID NO: 9, and
   VL-CDR3 shown in SEQ ID NO: 10;
   or
the heavy chain variable region comprises the following three complementary determining regions or CDRs:
   VH-CDR1 shown in SEQ ID NO: 13,
   VH-CDR2 shown in SEQ ID NO: 14, and
   VH-CDR3 shown in SEQ ID NO: 15;
the light chain variable region comprises the following three complementary determining regions or CDRs:
   VL-CDR1 shown in SEQ ID NO: 18,
   VL-CDR2 shown in SEQ ID NO: 19, and
   VL-CDR3 shown in SEQ ID NO: 20;
   or
the heavy chain variable region comprises the following three complementary determining regions or CDRs:
   VH-CDR1 shown in SEQ ID NO: 23,
   VH-CDR2 shown in SEQ ID NO: 24, and
   VH-CDR3 shown in SEQ ID NO: 25;
the light chain variable region comprises the following three complementary determining regions or CDRs:
   VL-CDR1 shown in SEQ ID NO: 28,
   VL-CDR2 shown in SEQ ID NO: 29, and
   VL-CDR3 shown in SEQ ID NO: 30;
   or
the heavy chain variable region comprises the following three complementary determining regions or CDRs:
   VH-CDR1 shown in SEQ ID NO: 33,
   VH-CDR2 shown in SEQ ID NO: 34, and
   VH-CDR3 shown in SEQ ID NO: 35;
the light chain variable region comprises the following three complementary determining regions or CDRs:
   VL-CDR1 shown in SEQ ID NO: 38,
   VL-CDR2 shown in SEQ ID NO: 39, and
   VL-CDR3 shown in SEQ ID NO: 40;
   or
the heavy chain variable region comprises the following three complementary determining regions or CDRs:
   VH-CDR1 shown in SEQ ID NO: 43,
   VH-CDR2 shown in SEQ ID NO: 44, and
   VH-CDR3 shown in SEQ ID NO: 45;
the light chain variable region comprises the following three complementary determining regions or CDRs:
   VL-CDR1 shown in SEQ ID NO: 48,
   VL-CDR2 shown in SEQ ID NO: 49, and
   VL-CDR3 shown in SEQ ID NO: 50.

In another preferred embodiment, the heavy chain variable region of the antibody has the amino acid sequence shown in SEQ ID NO: 1, 11, 21, 31, or 41; and/or the light chain variable region of the antibody has the amino acid sequence shown in SEQ ID NO: 6, 16, 26, 36, or 46.

In another preferred embodiment, the heavy chain variable region of the antibody has the amino acid sequence shown in SEQ ID NO: 1, and the light chain variable region of the antibody has the amino acid sequence shown in SEQ ID NO: 6.

In another preferred embodiment, the heavy chain variable region of the antibody has the amino acid sequence shown in SEQ ID NO: 11, and the light chain variable region of the antibody has the amino acid sequence shown in SEQ ID NO: 16.

In another preferred embodiment, the heavy chain variable region of the antibody has the amino acid sequence shown in SEQ ID NO: 21, and the light chain variable region of the antibody has the amino acid sequence shown in SEQ ID NO: 26.

In another preferred embodiment, the heavy chain variable region of the antibody has the amino acid sequence shown in SEQ ID NO: 31, and the light chain variable region of the antibody has the amino acid sequence shown in SEQ ID NO: 36.

In another preferred embodiment, the heavy chain variable region of the antibody has the amino acid sequence shown in SEQ ID NO: 41, and the light chain variable region of the antibody has the amino acid sequence shown in SEQ ID NO: 46.

In another preferred embodiment, the antibody is selected from the group consisting of:

| Antibody number | Clone | VH sequence number | VL sequence number |
|---|---|---|---|
| 1 | 147D7H2 | 1 | 6 |
| 2 | 102A2E11B6 | 11 | 16 |
| 3 | 69F2F3 | 21 | 26 |
| 4 | 154F10G8 | 31 | 36 |
| 5 | 151G3B11 | 41 | 46. |

In another preferred embodiment, the amino acid sequence of the heavy chain variable region has a sequence homology or a sequence identity of at least 80%, 85%, 90%, 91%, 92%, 93%, 94%, 95%, 96%, 97%, 98% or 99% with the amino acid sequence shown in SEQ ID NO: 1, 11, 21, 31, or 41 in the sequence listing.

In another preferred embodiment, the amino acid sequence of the light chain variable region has a sequence homology or a sequence identity of at least 80%, 85%, 90%, 91%, 92%, 93%, 94%, 95%, 96%, 97%, 98% or 99% with the amino acid sequence shown in SEQ ID NO: 6, 16, 26, 36, or 46 in the sequence listing.

In a sixth aspect of the present invention, it provides a recombinant protein, wherein the recombinant protein comprises:
(i) the heavy chain variable region according to the first aspect of the present invention, the heavy chain according to the second aspect of the present invention, the light chain variable region according to the third aspect of the present invention, the light chain according to the fourth aspect of the present invention, or the antibody according to the fifth aspect of the present invention; and
(ii) an optional tag sequence that assists expression and/or purification.

In another preferred embodiment, the tag sequence comprises a 6His tag.

In another preferred embodiment, the recombinant protein (or polypeptide) comprises fusion protein.

In another preferred embodiment, the recombinant protein is a monomer, a dimer, or a multimer.

In another preferred embodiment, the recombinant protein comprises
(i) an antibody selected from the group consisting of:

| Antibody number | Clone | VH sequence number | VL sequence number |
|---|---|---|---|
| 1 | 147D7H2 | 1 | 6 |
| 2 | 102A2E11B6 | 11 | 16 |
| 3 | 69F2F3 | 21 | 26 |
| 4 | 154F10G8 | 31 | 36 |
| 5 | 151G3B11 | 41 | 46 |

and
(ii) an optional tag sequence that assists expression and/or purification.

In a seventh aspect of the present invention, it provides a polynucleotide, which encodes a polypeptide selected from the group consisting of:
(i) the heavy chain variable region according to the first aspect of the present invention, the heavy chain according to the second aspect of the present invention, the light chain variable region according to the third aspect of the present invention, the light chain according to the fourth aspect of the present invention, or the antibody according to the fifth aspect of the present invention; and
   (2) the recombinant protein according to the sixth aspect of the present invention.

In another preferred embodiment, the polynucleotide encoding the heavy chain variable region is as shown in SEQ ID NO: 2, 12, 22, 32, or 42; and/or, the polynucleotide encoding the light chain variable region is as shown in SEQ ID NO: 7, 17, 27, 37, or 47.

In another preferred embodiment, the polynucleotide encoding the heavy chain variable region sequence and the polynucleotide encoding the light chain variable region sequence are selected from the group consisting of:

| Clone | Sequence number of the polynucleotide encoding VH | Sequence number of the polynucleotide encoding VL |
|---|---|---|
| 147D7H2 | 2 | 7 |
| 102A2E11B6 | 12 | 17 |
| 69F2F3 | 22 | 27 |
| 154F10G8 | 32 | 37 |
| 151G3B11 | 42 | 47. |

In an eighth aspect of the present invention, it provides a vector, which comprises the polynucleotide according to the seventh aspect of the present invention.

In another preferred embodiment, the vector comprises: a bacterial plasmid, a phage, a yeast plasmid, a plant cell virus, a mammalian cell virus such as an adenovirus, retrovirus, or other vectors.

In a ninth aspect of the present invention, it provides a genetically engineered host cell, wherein the host cell comprises the vector according to the eighth aspect of the present invention or the genome thereof is integrated with the polynucleotide according to the seventh aspect of the present invention..

In a tenth aspect of the present invention, it provides an antibody conjugate, which comprises:
(i) an antibody moiety, which is selected from the group consisting of: the heavy chain variable region according to the first aspect of the present invention, the heavy chain according to the second aspect of the present invention, the light chain variable region according to the third aspect of the present invention, the light chain according to the fourth aspect of the present invention, and the antibody according to the fifth aspect of the present invention, and a combination thereof; and
   (b) a coupling moiety coupled to the antibody moiety, and the coupling moiety is selected from the group consisting of a detectable label, a drug, a toxin, a cytokine, a radionuclide, an enzyme, and a combination thereof.

In another preferred embodiment, the antibody moiety is coupled to the coupling moiety via a chemical bond or linker.

In an eleventh aspect of the present invention, it provides an immune cell, which expresses or is exposed outside the cell membrane with the antibody according to the fifth aspect of the present invention.

In another preferred embodiment, the immune cell includes NK cells and T cells.

In another preferred embodiment, the immune cell is derived from human or non-human mammals (such as mice).

In a twelfth aspect of the present invention, it provides a pharmaceutical composition, wherein the pharmaceutical composition comprises:
(i) an active ingredient, wherein the active ingredient is selected from the group consisting of: the heavy chain variable region according to the first aspect of the present invention, the heavy chain according to the second aspect of the present invention, the light chain variable region according to the third aspect of the present invention, the light chain according to the fourth aspect of the present invention, and the antibody according to the fifth aspect of the present invention, the recombinant protein according to the sixth aspect of the present invention, the antibody conjugate according to the tenth aspect of the present invention, the immune cell according to the eleventh aspect of the present invention, and combinations thereof; and
(ii) a pharmaceutically acceptable carrier.

In another preferred embodiment, the pharmaceutical composition is a liquid formulation.

In another preferred embodiment, the pharmaceutical composition is an injection.

In another preferred embodiment, the pharmaceutical composition comprises 0.01-99.99% of the antibody according to the fifth aspect of the present invention, the recombinant protein according to the sixth aspect of the present invention, the antibody conjugate according to the tenth aspect of the present invention, the immune cell according to the eleventh aspect of the present invention, and a combination thereof, and 0.01-99.99% of the pharmaceutically acceptable carrier, wherein the percentage is the mass percentage of the pharmaceutical composition.

In a thirteenth aspect of the present invention, it provides use of an active ingredient, wherein the active ingredient is selected from the group consisting of: the heavy chain variable region according to the first aspect of the present invention, the heavy chain according to the second aspect of the present invention, the light chain variable region according to the third aspect of the present invention, the light chain according to the fourth aspect of the present invention, the antibody according to the fifth aspect of the present invention, the recombinant protein according to the sixth aspect of the present invention, the antibody conjugate according to the tenth aspect of the present invention, the immune cell according to the eleventh aspect of the present invention, and combinations thereof, wherein the active ingredient is used for (a) preparation of a diagnostic reagent or kit; and/or (b) preparation of a medicine for preventing and/or treating diseases associated with abnormal IL-5 expression or function.

In another preferred embodiment, the diagnostic reagent is a detection piece or a detection plate.

In another preferred embodiment, the disease associated with abnormal IL-5 expression or function is selected from the group consisting of: asthma, atopic dermatitis, and inflammatory diseases.

In another preferred embodiment, the inflammatory disease is chronic obstructive pulmonary disease.

In another preferred embodiment, the diagnostic reagent or kit is used for:
(1) detecting IL-5 protein in a sample; and/or
(2) detecting endogenous IL-5 protein in cells; and/or
(3) detecting cells expressing IL-5 protein.
the medicine is used for preventing and/or treating diseases associated with abnormal IL-5 expression or function, and the diseases associated with abnormal IL-5 expression or function is asthma, atopic dermatitis, and inflammatory diseases.

In another preferred embodiment, the inflammatory disease is chronic obstructive pulmonary disease.

In another preferred embodiment, the antibody is in the form of a drug conjugate (ADC).

In another preferred embodiment, the diagnostic reagent or kit is used for diagnosis of IL-5 related diseases.

In another preferred embodiment, the diagnostic reagent or kit is used for detection of IL-5 protein in a sample.

In a fourteenth aspect of the present invention, it provides a method for *in vitro* detection (including diagnostic or non-diagnostic) of IL-5 protein in a sample, wherein the method comprises the steps:
(1) contacting the sample with the antibody according to the fifth aspect of the present invention *in vitro;*
(2) detecting whether an antigen-antibody complex is formed, wherein the formation of a complex indicates the presence of IL-5 protein in the sample.

In a fifteenth aspect of the present invention, it provides a composition for detecting IL-5 protein in a sample *in vitro,* which comprises the antibody according to the fifth aspect of the present invention, the recombinant protein according to the sixth aspect of the present invention, the antibody conjugate according to the tenth aspect of the present invention, the immune cell according to the eleventh aspect of the present invention, and a combination thereof, as an active ingredient.

In a sixteenth aspect of the present invention, it provides a detection plate, wherein the detection plate comprises: a substrate (support plate) and a detection strip, wherein the detection strip comprises the antibody according to the fifth aspect of the present invention, the recombinant protein according to the sixth aspect of the present invention, the antibody conjugate according to the tenth aspect of the present invention, the immune cell according to the eleventh aspect of the present invention, and a combination thereof.

In a seventeenth aspect of the present invention, it provides a kit, which comprises:
(1) a first container, which contains the antibody of the present invention; and/or
(2) a second container, which contains a secondary antibody against the antibody of the present invention;
or,
the kit comprises the detection plate according to the sixteenth aspect of the present invention.

In an eighteenth aspect of the present invention, it provides a method for preparing a recombinant polypeptide, wherein the method comprises:
(a) culturing the host cell according to the ninth aspect of the present invention under conditions suitable for expression;
(b) isolating a recombinant polypeptide from the culture, wherein the recombinant polypeptide is the antibody according to the fifth aspect of the present invention or the recombinant protein according to the sixth aspect of the present invention.

In a nineteenth aspect of the present invention, it provides a drug combination, comprising:
(i) a first active ingredient, which comprises the antibody according to the fifth aspect of the present invention, or the recombinant protein according to the sixth aspect of the present invention, or the antibody conjugate according to the tenth aspect of the present invention, or the immune cell according to the eleventh aspect of the present invention, or the pharmaceutical composition according to the twelfth aspect of the present invention, and a combination thereof;
(ii) a second active ingredient, which comprises a second antibody, or a chemotherapeutic agent.

In another preferred embodiment, the second antibody is selected from the group consisting of an IL-13 antibody and an IL-4 antibody.

In another preferred embodiment, the second antibody is an IL-13 antibody.

In another preferred example, the chemotherapeutic agent is selected from the group consisting of docetaxel, carboplatin, and a combination thereof.

In a twentieth aspect of the present invention, it provides use of a combination for preparation of a medicine for the treatment of diseases associated with abnormal IL-5 expression or function, wherein the combination comprises the antibody according to the fifth aspect of the present invention, or the recombinant protein according to the sixth aspect of the present invention, or the antibody conjugate according to the tenth aspect of the present invention, or the immune cell according to the eleventh aspect of the present invention, and/or the pharmaceutical composition according to the twelfth aspect of the present invention, as well as a second antibody or a chemotherapeutic agent.

In another preferred embodiment, the second antibody is selected from the group consisting of an IL-13 antibody and an IL-4 antibody.

In another preferred embodiment, the second antibody is an IL-13 antibody.

In a twenty-first aspect of the present invention, it provides a method for the treatment of diseases associated with abnormal IL-5 expression or function, which comprises administering an effective amount of the antibody according to the fifth aspect of the present invention, the recombinant protein according to the sixth aspect of the present invention, the antibody conjugate according to the tenth aspect of the present invention, the immune cell according to the eleventh aspect of the present invention, the pharmaceutical composition of the twelfth aspect of the present invention, and a combination thereof, to a subject in need.

In another preferred embodiment, the disease associated with abnormal IL-5 expression or function is asthma, atopic dermatitis, or inflammation diseases.

In another preferred embodiment, the inflammatory disease is chronic obstructive pulmonary disease.

In another preferred embodiment, the method further comprises: administering a safe and effective amount of a second antibody to the subject before, during, and/or after administering the first active ingredient.

In another preferred embodiment, the second antibody is selected from the group consisting of an IL-13 antibody and an IL-4 antibody.

In another preferred embodiment, the second antibody is an IL-13 antibody.

It should be understood that the various technical features of the present invention mentioned above and the various technical features specifically described hereinafter (as in the Examples) may be combined with each other within the scope of the present invention to constitute a new or preferred technical solution, which need not be described one by one, due to space limitations.

### Description of Drawings

Figure 1 shows the biological activity of recombinant IL-5 protein.
Figure 2 shows the serum antibody titer of mice immunized with recombinant IL-5 protein detected by ELISA.
Figure 3 shows the reactivity of IL-5 antibody with human IL-5 in ELISA (enzyme linked immunosorbent assay).
Figure 4 shows the reactivity of IL-5 antibody with monkey IL-5 in ELISA (enzyme linked immunosorbent assay).
Figure 5 shows the reactivity of IL-5 antibody with mouse IL-5 in ELISA (enzyme-linked immunosorbent assay).
Figure 6 shows the expression level of IL5Ra/CSF2RB protein in CHOK1 recombinant cell line detected by flow cytometry.
Figure 7 shows the blocking of IL-5 antibody on the binding of IL-5 to cell surface receptor IL-5Ra detected by flow cytometry.
Figure 8 shows the neutralization of IL-5 antibody against CTLL-2 proliferation experiment stimulated by IL-5.
Figure 9 shows the neutralization of IL-5 antibody against TF-1 proliferation experiment stimulated by IL-5.
Figure 10 shows the affinity test of IL-5 antibody and human IL-5.
Figure 11 shows the reactivity of IL-5 chimeric antibody with human IL-5 in ELISA (enzyme-linked immunosorbent assay)
Figure 12 shows the reactivity of IL-5 chimeric antibody with monkey IL-5 in ELISA (enzyme-linked immunosorbent assay).
Figure 13 show the reactivity of IL-5 chimeric antibody with mouse IL-5 in ELISA (enzyme-linked immunosorbent assay).
Figure 14 shows the blocking of IL-5 chimeric antibody on the binding of IL-5 to cell surface receptor IL-5Ra detected by flow cytometry.
Figure 15 shows the neutralization of IL-5 chimeric antibody against CTLL-2 proliferation experiment stimulated by IL-5.
Figure 16 shows the neutralization of IL-5 chimeric antibody against TF-1 proliferation experiment stimulated by IL-5.
Figure 17 shows the affinity test of IL-5 chimeric antibody and human IL-5.

### Modes for carrying out the present invention

Through extensive and intensive research, the inventors prepared a group of specific monoclonal murine antibodies binding to IL-5 by optimized hybridoma technology using human IL-5 as immunogen. The DNA and amino acid sequences of the variable region were determined by molecular biological methods. The variable regions of the murine antibody were combined with the constant region of human antibody to form a mouse-human chimeric antibody molecule, or converted into a humanized antibody molecule through humanization technology, or constructed into other molecular forms such as bispecific antibodies, multispecific antibodies, single chain antibodies, single fragment antibodies, etc. according to specific purposes. These antibodies can bind to human IL-5 protein and inhibit the binding of IL-5 to its receptor IL-5Ra subunit. These antibodies show good biological activity in experiments that neutralize the proliferation of CTLL-2 and TF-1 stimulated by IL-5. The anti-IL-5 antibody exerts the function of inhibiting the proliferation and differentiation of eosinophils by IL-5, which can well relieve a series of asthma symptoms caused by IL-5, thereby playing the role of treating asthma. Especially for severe asthma, it can play an auxiliary role in conventional treatment or even replace conventional therapy. In addition, the present invention also provides the use of these antibodies, including but not limited to the use of neutralizing IL-5 to reduce the differentiation and proliferation of eosinophils, i.e. to reduce the accumulation and infiltration of eosinophils and other asthmatic symptoms, the use alone or in combination with other anti-asthmatic antibodies for the treatment of asthma, and the associated use for the diagnosis of asthmatic patients. The present invention has been completed on the basis of this.

### Terms

In the present invention, "VH-CDR1" and "CDR-H1" can be used interchangeably and both refer to CDR1 of heavy chain variable region; "VH-CDR2" and "CDR-H2" can be used interchangeably and both refer to CDR2 of heavy chain variable region; "VH-CDR3" and "CDR-H3" can be used interchangeably and both refer to CDR3 of heavy chain variable region. "VL-CDR1" and "CDR-L1" can be used interchangeably and both refer to CDR1 of light chain variable region; "VL-CDR2" and "CDR-L2" can be used interchangeably and both refer to CDR2 of light chain variable region; "VL-CDR3" and "CDR-L3" can be used interchangeably and both refer to CDR3 of light chain variable region.

### Antibody

As used herein, the term "antibody" or "immunoglobulin" is a heterotetrameric glycoprotein of about 150,000 Da having the same structural characteristics, which consists of two identical light chains (L) and two identical heavy chains (H). Each light chain is linked to a heavy chain via a covalent disulfide bond, and different immunoglobulin isotypes have different numbers of disulfide bonds between the heavy chains. There are also regularly spaced intrachain disulfide bonds in each heavy and each light chain. Each heavy chain has a variable region (VH) at one end, followed by a plurality of constant regions. Each light chain has a variable region (VL) at one end and a constant region at the other end; the constant region of a light chain pairs with the first constant region of a heavy chain, and the variable region of a light chain pairs with the variable region of a heavy chain. Special amino acid residues form an interface between the variable regions of a light chain and a heavy chain.

As used herein, the term "variable" means that antibodies are different from each other in terms of sequence in certain parts of variable regions, which is responsible for the binding and specificity of various specific antibodies to their specific antigens. However, the variability is not distributed evenly throughout the variable regions of an antibody. It is concentrated in three segments called complementary determining regions (CDRs) or hypervariable regions in the light and heavy chain variable regions. The conserved parts of variable regions are called framework regions (FRs). Each of the variable regions of naturally occurring heavy and light chains comprises four FR regions, which are generally in a β-sheet configuration, joined by the three CDRs forming a linking loop, and in some cases, may form a partial β-sheet structure. The CDRs in each chain are closely linked together via the FR regions, and together with the CDRs of the other chain, form the antigen binding site of an antibody (see Kabat et al., NIH Publ. No. 91-3242, Vol. I, pp. 647-669 (1991)). The constant regions are not directly involved in the binding of an antibody to an antigen, however, they exhibit different effector functions, for example, involved in the antibody-dependent cytotoxicitiy of an antibody.

The "light chain" of a vertebrate antibody (immunoglobulin) can be classified into one of the two obviously different classes (referred to as κ and λ) depending on the amino acid sequence of its constant region. Immunoglobulins can be classified into different classes depending on the amino acid sequences of their heavy chain constant regions. There are mainly five classes of immunoglobulins: IgA, IgD, IgE, IgG, and IgM, some of which can be further classified into subclasses (isotypes), such as IgG1, IgG2, IgG3, IgG4, IgA, and IgA2. The heavy chain constant regions corresponding to different classes of immunoglobulins are called α, δ, ε, γ, and µ, respectively. The subunit structures and three-dimensional configurations of different classes of immunoglobulins are well known for those skilled in the art.

In general, the antigen binding characteristics of an antibody can be described by three specific regions located in the heavy and light chain variable regions, called complementary determining regions (CDRs), which divide the variable region into four framework regions (FRs); the amino acid sequences of the four FRs are relatively conservative and are not directly involved in the binding reaction. These CDRs form a ring structure, and approach to each other in the steric structure by virtue of the β-sheets formed by the FRs between them, and the CDRs on the heavy chain and the CDRs on the corresponding light chain constitute the antigen-binding site of an antibody. By comparison of the amino acid sequences of antibodies of the same type, it can be determined which amino acids form FRs or CDRs.

The present invention includes not only an intact antibody, but also the fragments of the antibody having an immunological activity or a fusion protein formed by the antibody and another sequence. Therefore, the present invention also includes fragments, derivatives and analogs of the antibody.

In the present invention, antibodies include murine, chimeric, humanized or fully human antibodies as prepared by techniques well known to those skilled in the art. Recombinant antibodies, such as chimeric and humanized monoclonal antibodies, including human and non-human portions, can be obtained by standard DNA recombination techniques, all of which are useful antibodies. A chimeric antibody is a molecule in which different portions are derived from different animal species, for example, a chimeric antibody having a variable region from a monoclonal antibody from a mouse and a constant region from a human immunoglobulin (see, for example, U.S. Pat. Nos. 4,816,567 and 4,816,397, which are incorporated herein by reference in its entirety). A humanized antibody refers to an antibody molecule derived from a non-human species, which has one or more complementary determining regions (CDRs) derived from a non-human species and framework regions derived from a human immunoglobulin molecule (see U.S. Pat. No. 5,585,089, which is incorporated herein by reference in its entirety). These chimeric and humanized monoclonal antibodies can be prepared by recombinant DNA techniques well known in the art.

In the present invention, an antibody may be monospecific, bispecific, trispecific, or multispecific.

In the present invention, the antibody of the present invention further includes a conservative variant thereof, which refers to a polypeptide formed by substitution of at most 10, preferably at most 8, more preferably at most 5, and most preferably at most 3 amino acids with amino acids having similar or analogous property, as compared to the amino acid sequence of the antibody of the present invention. These conservative variant polypeptides are preferably formed by carrying out the amino acid substitution according to Table 23.

**Table 23**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

### Anti-IL-5 antibody

In the present invention, the antibody (antibody of the present invention) is an anti-IL-5 antibody. The present invention provides an IL-5 targeting antibody with high specificity and high affinity, which comprises a heavy chain and a light chain. The heavy chain comprises the amino acid sequence of heavy chain variable region (VH), and the light chain comprises the amino acid sequence of light chain variable region (VL).

Preferably,
the heavy chain variable region (VH) comprises complementary determining regions or CDRs selected from the group consisting of:
   VH-CDR1 shown in SEQ ID NO: 10n+3,
   VH-CDR2 shown in SEQ ID NO: 10n+4, and
   VH-CDR3 shown in SEQ ID NO: 10n+5;
   wherein each n is independently 0, 1, 2, 3, or 4;
the light chain variable region (VL) comprises complementary determining regions or CDRs selected from the group consisting of:
   VL-CDR1 shown in SEQ ID NO: 10n+8,
   VL-CDR2 shown in SEQ ID NO: 10n+9, and
   VL-CDR3 shown in SEQ ID NO: 10n+10;
      wherein each n is independently 0, 1, 2, 3, or 4;
      wherein any one of the above amino acid sequences further comprises a derivative sequence which is obtained through optional addition, deletion, modification and/or substitution of at least one amino acid and is capable of retaining the binding affinity to IL-5.

Preferably, the heavy chain variable region (VH) comprises the following three complementary determining regions or CDRs:
VH-CDR1 shown in SEQ ID NO: 10n+3,
VH-CDR2 shown in SEQ ID NO: 10n+4, and
VH-CDR3 shown in SEQ ID NO: 10n+5;
the light chain variable region (VL) comprises the following three complementary determining regions or CDRs:
VL-CDR1 shown in SEQ ID NO: 10n+8,
VL-CDR2 shown in SEQ ID NO: 10n+9, and
VL-CDR3 shown in SEQ ID NO: 10n+10;
   wherein each n is independently 0, 1, 2, 3, or 4; preferably n is 0 or 1;
   wherein any one of the above amino acid sequences further comprises a derivative sequence which is obtained through optional addition, deletion, modification and/or substitution of at least one amino acid and is capable of retaining the binding affinity to IL-5.

In another preferred embodiment, the sequence with at least one amino acid added, deleted, modified and/or substituted in any of the above amino acid sequences is preferably an amino acid sequence having a homology or sequence identity of at least 80%, preferably at least 85%, more preferably at least 90%, most preferably at least 95% to the above amino acid sequence.

Method for determining sequence homology or identity that are well known to the ordinary skilled in the art includes, but are not limited to: Computer Molecular Biology, edited by Lesk, A.M., Oxford University Press, New York, 1988; Biocomputing; Biocomputing: Informatics and Genome Projects, edited by Smith, D.W., Academic Press, New York, 1993; Computer Analysis of Sequence Data, Part I, edited by Griffin, A.M. and Griffin, H.G., Humana Press, New Jersey, 1994; Sequence Analysis in Molecular Biology, von Heinje, G., Academic Press, 1987, and Sequence Analysis Primer, edited by Gribskov, M. and Devereux, J., M Stockton Press, New York, 1991 and Carillo, H. & Lipman, D., SIAM J. Applied Math ., 48:1073(1988). The preferred method for determining identity is to obtain the greatest match between the sequences tested. Methods for determining identity are compiled into publicly available computer programs. Preferred computer program method for determining identity between two sequences includes, but are not limited to, the GCG software package (Devereux, J. et al., 1984), BLASTP, BLASTN, and FASTA (Altschul, S, F. et al., 1990). The BLASTX program is available to the public from NCBI and other sources (BLAST Handbook, Altschul, S. et al., NCBI NLM NIH Bethesda, Md. 20894; Altschul, S. et al., 1990). The well-known Smith Waterman algorithm can also be used to determine identity.

The antibody in the present invention can be a full-length protein (such as IgG1, IgG2 (for example IgG2a, IgG2b or IgG2c), IgG3 or IgG4), or a protein fragment containing an antigen-antibody binding domain (such as Fab, F(ab'), sdAb, ScFv fragments). The antibody in the present invention can be a wild-type protein, or a mutant protein that has achieved a certain effect through specific mutations, for example, using mutations to eliminate the effector function of the antibody.

Preferably, the antibody described herein is one or more of an antibody full-length protein, an antigen-antibody binding domain protein fragment, a bispecific antibody, a multispecific antibody, a single chain antibody fragment (scFv), a single domain antibody (sdAb) and a Single-domain antibody, as well as a monoclonal antibody or a polyclonal antibody made from the above antibodies. The monoclonal antibody can be developed by a variety of approaches and technologies, including hybridoma technology, phage display technology, single lymphocyte gene cloning technology, etc. The mainstream is to prepare monoclonal antibodies from wild-type or transgenic mice through hybridoma technology.

The antibody full-length protein is a conventional antibody full-length protein in the art, which comprises a heavy chain variable region, a light chain variable region, a heavy chain constant region, and a light chain constant region. The heavy chain variable region and light chain variable region of the protein and human heavy chain constant region and human light chain constant region constitute a fully human antibody full-length protein. Preferably, the antibody full-length protein is IgG1, IgG2, IgG3 or IgG4.

The antibody of the present invention may be a double-chain or single-chain antibody, and may be selected from animal-derived antibodies, chimeric antibodies and humanized antibodies, more preferably be selected from humanized antibodies and human-animal chimeric antibodies, more preferably a fully humanized antibody.

The antibody derivative of the present invention may be a single-chain antibody, and/or an antibody fragment, for example, Fab, Fab', (Fab')2 or other antibody derivatives known in the art, etc., and may be any one or more of IgA, IgD, IgE, IgG and IgM antibodies or other subtype antibodies.

The single-chain antibody is a conventional single-chain antibody in the art, which comprises a heavy chain variable region, a light chain variable region and a short peptide of 15-20 amino acids.Wherein, the animal is preferably a mammal, such as mouse.

The antibody of the present invention may be a chimeric antibody, a humanized antibody, a CDR grafted and/or modified antibody that targets IL-5, such as human IL-5.

In above content of the present invention, the number of the added, deleted, modified and/or substituted amino acids, preferably does not exceed 40%, more preferably does not exceed 35%, is more preferably 1-33%, is more preferably 5-30%, is more preferably 10-25%, and is more preferably 15-20% of the total number of the amino acids of the initial amino acid sequence.

In the above content of the present invention, more preferably, the number of the added, deleted, modified and/or substituted amino acids, may be 1-7, more preferably 1-5, more preferably 1-3, and more preferably 1-2.

In another preferred embodiment, the heavy chain variable region of the antibody has the amino acid sequence shown in SEQ ID NO: 1, 11, 21, 31, or 41.

In another preferred embodiment, the light chain variable region of the antibody has the amino acid sequence shown in SEQ ID NO: 6, 16, 26, 36, or 46.

In another preferred embodiment, the amino acid sequences of the heavy chain variable region and/or the light chain variable region of the antibody targeting IL-5 are shown in the following Table 24:

**Table 24**

| Antibody number | VH sequence number | VL sequence number |
|---|---|---|
| 1 | 1 | 6 |
| 2 | 11 | 16 |
| 3 | 21 | 26 |
| 4 | 31 | 36 |
| 5 | 41 | 46. |

In another preferred embodiment, the antibody targeting IL-5 is 147D7H2, 102A2E11B6, 69F2F3, 154F10G8, 151G3B11, 100F4E11B3, 144D3C4A5, 90F9G10H2, 136E11F2, or 128G11B3.

In another preferred embodiment, the antibody targeting IL-5 is 147D7H2.

In another preferred embodiment, the antibody targeting IL-5 is 102A2E11B6.

### Recombinant protein

The invention also provides a recombinant protein comprising one or more of heavy chain CDR1 (VH-CDR1), heavy chain CDR2 (VH-CDR2) and heavy chain CDR3 (VH-CDR3) of an IL-5 antibody, and/or one or more of light chain CDR1 (VL-CDR1), light chain CDR2 (VL-CDR2) and light chain CDR3 (VL-CDR3) of an IL-5 antibody,

The sequences of the heavy chain CDR1-3 are as follows:
VH-CDR1 shown in SEQ ID NO: 10n+3,
VH-CDR2 shown in SEQ ID NO: 10n+4,
VH-CDR3 shown in SEQ ID NO: 10n+5;

The sequences of the light chain CDR1-3 are as follows:
VL-CDR1 shown in SEQ ID NO: 10n+8,
VL-CDR2 shown in SEQ ID NO: 10n+9, and
VL-CDR3 shown in SEQ ID NO: 10n+10;
   wherein each n is independently 0, 1, 2, 3, or 4; preferably n is 0 or 1;
   wherein any one of the above amino acid sequences further comprises a derivative sequence which is obtained through optional addition, deletion, modification and/or substitution of at least one amino acid and is capable of retaining the binding affinity to IL-5.

In another preferred embodiment, the sequence with at least one amino acid added, deleted, modified and/or substituted in any of the above amino acid sequences is preferably an amino acid sequence having a homology or sequence identity of at least 80%, more preferably at least 85%, more preferably at least 90%, most preferably at least 95% to the above amino acid sequence.

In another preferred embodiment, the recombinant protein according to the present invention comprises a heavy chain variable region of an IL-5 antibody and/or a light chain variable region of an IL-5 antibody, the heavy chain variable region of the antibody has the amino acid sequence shown in SEQ ID NO: 1, 11, 21, 31, or 41; and the light chain variable region of the antibody has the amino acid sequence shown in SEQ ID NO: 6, 16, 26, 36, or 46.

In another preferred embodiment, the recombinant protein according to the present invention comprises a heavy chain variable region of an IL-5 antibody and a light chain variable region of an IL-5 antibody, the heavy chain variable region of the antibody has the amino acid sequence shown in SEQ ID NO: 1, 11, 21, 31, or 41; and the light chain variable region of the antibody has the amino acid sequence shown in SEQ ID NO: 6, 16, 26, 36, or 46.

In another preferred embodiment, the amino acid sequence numbers of the recombinant protein as well as the heavy chain CDR1-3 and light chain CDR1-3 comprised therein are as shown in Table 25:

**Table 25 Amino acid sequence numbers of heavy chain CDR1-3 and light chain CDR1-3**

| Recombinant protein numbers | Heavy chain protein | | | | Light chain protein | | | |
|---|---|---|---|---|---|---|---|---|
| | Variable region | VH-CDR1 | VH-CDR2 | VH-CDR3 | Variable region | VL-CDR1 | VL-CDR2 | VL-CDR3 |
| 1 | 1 | 3 | 4 | 5 | 6 | 8 | 9 | 10 |
| 2 | 11 | 13 | 14 | 15 | 16 | 18 | 19 | 20 |
| 3 | 21 | 23 | 24 | 25 | 26 | 28 | 29 | 30 |
| 4 | 31 | 33 | 34 | 35 | 36 | 38 | 39 | 40 |
| 5 | 41 | 43 | 44 | 45 | 46 | 48 | 49 | 50 |

wherein any one of the above amino acid sequences further comprises a derivative sequence which is obtained through optional addition, deletion, modification and/or substitution of at least one amino acid and is capable of retaining the binding affinity to IL-5.

Preferably, the recombinant protein further comprises an antibody heavy chain constant region and/or an antibody light chain constant region, wherein the antibody heavy chain constant region is conventional in the art, preferably a rat antibody heavy chain constant region or a human antibody heavy chain constant region, more preferably a human antibody heavy chain constant region. The antibody light chain constant region is conventional in the art, preferably a rat antibody light chain constant region or a human antibody light chain constant region, more preferably a human antibody light chain constant region.

The recombinant protein is a conventional protein in the art. Preferably, it is one or more of an antibody full-length protein, an antigen-antibody binding domain protein fragment, a bispecific antibody, a multispecific antibody, a single chain antibody fragment (scFv), a single domain antibody (sdAb) and a single-domain antibody, as well as a monoclonal antibody or a polyclonal antibody made from the above antibodies. The monoclonal antibody can be developed by a variety of approaches and technologies, including hybridoma technology, phage display technology, single lymphocyte gene cloning technology, etc. The mainstream is to prepare monoclonal antibodies from wild-type or transgenic mice through hybridoma technology.

The antibody full-length protein is a conventional antibody full-length protein in the art, which comprises a heavy chain variable region, a light chain variable region, a heavy chain constant region, and a light chain constant region. The heavy chain variable region and light chain variable region of the protein and human heavy chain constant region and human light chain constant region constitute a fully human antibody full-length protein. Preferably, the antibody full-length protein is IgG1, IgG2, IgG3 or IgG4.

The single-chain antibody is a conventional single-chain antibody in the art, which comprises a heavy chain variable region, a light chain variable region and a short peptide of 15-20 amino acids.

The antigen-antibody binding domain protein fragments are conventional antigen-antibody binding domain protein fragments in the art, which comprise a light chain variable region, a light chain constant region, and an Fd segment of heavy chain constant region. Preferably, the antigen-antibody binding domain protein fragments are Fab and F (ab').

The single domain antibody is a conventional single domain antibody in the art, which comprises a heavy chain variable region and a heavy chain constant region.

The single-domain antibody is a conventional single-domain antibody in the art, which only comprises a heavy chain variable region.

Wherein, the preparation method of the recombinant protein is a conventional preparation method in the art. Preferably, the preparation method is: isolating and obtaining the protein from an expression transformant that recombinantly expresses the protein or obtaining the protein by artificially synthesizing a protein sequence. The method of isolating and obtaining the protein from an expression transformant that recombinantly expresses the protein is preferably as follows: cloning a nucleic acid molecule encoding the protein carrying a point mutation into a recombinant vector, and transforming the obtained recombinant vector into a transformant to obtain a recombinant expression transformant, and by culturing the obtained recombinant expression transformant, the recombinant protein can be obtained by separation and purification.

### Nucleic Acid

The present invention also provides a nucleic acid encoding the above-mentioned antibody (e.g., an anti-IL-5 antibody) or recombinant protein, or the heavy chain variable region or the light chain variable region of the anti-IL-5 antibody.

Preferably, the nucleotide sequence of the nucleic acid encoding the heavy chain variable region is shown in SEQ ID NO: 2, 12, 22, 32, or 42 in the sequence listing; and/or, the nucleotide sequence of the nucleic acid encoding the light chain variable region is shown in SEQ ID NO: 7, 17, 27, 37, or 47 in the sequence listing.

Preferably, the nucleotide sequence of the nucleic acid encoding the heavy chain variable region is shown in SEQ ID NO: 2, 12, 22, 32, or 42 in the sequence listing; and the nucleotide sequence of the nucleic acid encoding the light chain variable region is shown in SEQ ID NO: 7, 17, 27, 37, or 47 in the sequence listing.

The preparation method of the nucleic acid is a conventional preparation method in the art. Preferably, it comprises the following steps: obtaining the nucleic acid molecule encoding the above-mentioned protein by gene cloning technology, or obtaining the nucleic acid molecule encoding the above-mentioned protein by the method of artificial full-length sequence synthesis.

Those skilled in the art know that the base sequence encoding the amino acid sequence of the protein can be replaced, deleted, changed, inserted or added appropriately to provide a polynucleotide homolog. The homolog of the polynucleotide of the present invention can be prepared by replacing, deleting or adding one or more bases of the gene encoding the protein sequence within the scope of maintaining the activity of the antibody.

### Vector

The present invention also provides a recombinant expression vector comprising the nucleic acid.

The recombinant expression vector can be obtained by conventional methods in the art, that is, by connecting the nucleic acid molecule of the present invention to various expression vectors, thus being constructed. The expression vector is one of a variety of conventional vectors in the art, as long as it can carry the above-mentioned nucleic acid molecule. The vector preferably includes: various plasmids, cosmids, phage or virus vectors and the like.

The present invention also provides a recombinant expression transformant comprising the above-mentioned recombinant expression vector.

Wherein, the preparation method of the recombinant expression transformant is a conventional preparation method in the art, preferably comprising: being obtained by transforming the recombinant expression vector into a host cell. The host cell is one of a variety of conventional host cells in the art, as long as the recombinant expression vector can replicate itself stably and the nucleic acid carried can be effectively expressed. Preferably, the host cell is *E.coli* TGI or *E.coli* BL21 cell (for expressing single-chain antibodies or Fab antibodies), or HEK293 or CHO cell (for expressing full-length IgG antibodies). The above-mentioned recombinant expression plasmid is transformed into a host cell to obtain the preferred recombinant expression transformant of the present invention. The transformation method is a conventional transformation method in the art, preferably a chemical transformation method, a heat shock method or an electrotransformation method.

### Antibody preparation

The sequence of the DNA molecule for the antibody or a fragment thereof according to the present invention can be obtained by conventional techniques, for example, methods such as PCR amplification or genomic library screening. In addition, the sequences encoding light chain and heavy chain can be fused together, to form a single-chain antibody.

Once a relevant sequence is obtained, the relevant sequence can be obtained in bulk using a recombination method. This is usually carried out by cloning the sequence into a vector, transforming a cell with the vector, and then separating the relevant sequence from the proliferated host cell by conventional methods.

In addition, a relevant sequence can be synthesized artificially, especially when the fragment is short in length. Usually, several small fragments are synthesized first, and then are linked together to obtain a fragment with a long sequence.

At present, it is possible to obtain a DNA sequence encoding the antibody of the present invention (or fragments thereof, or derivatives thereof) completely by chemical synthesis. The DNA sequence can then be introduced into a variety of existing DNA molecules (or, for example, vectors) and cells known in the art. In addition, mutations can also be introduced into the protein sequences of the present invention by chemical synthesis.

The present invention further relates to a vector comprising said suitable DNA sequence and a suitable promoter or a control sequence. These vectors can be used to transform suitable host cells to enable them to express protein.

The host cell can be a prokaryotic cell, such as a bacterial cell; or a lower eukaryotic cell, such as a yeast cell; or a higher eukaryotic cell, such as a mammalian cell. Preferred animal cells include, but are not limited to, CHO-S, HEK-293 cells.

In general, under conditions suitable for expression of the antibody according to the present invention, the host cell obtained is cultured. Then, the antibody of the present invention is purified by using conventional immunoglobulin purification steps, for example, the conventional separation and purification means well known to those skilled in the art, such as protein A-Sepharose, hydroxyapatite chromatography, gel electrophoresis, dialysis, ion exchange chromatography, hydrophobic chromatography, molecular sieve chromatography or affinity chromatography.

The monoclonal antibody obtained can be identified by conventional means. For example, the binding specificity of a monoclonal antibody can be determined by immunoprecipitation or an *in vitro* binding assay (such as radioimmunoassay (RIA) or enzyme-linked immunosorbent assay (ELISA)). The binding affinity of a monoclonal antibody can be determined by, for example, the Scatchard analysis (Munson et al., Anal. Biochem., 107: 220 (1980)).

The antibody according to the present invention can be expressed in a cell or on the cell membrane, or is secreted extracellularly. If necessary, the recombinant protein can be separated and purified by various separation methods according to its physical, chemical, and other properties. These methods are well known to those skilled in the art. The examples of these methods comprise, but are not limited to, conventional renaturation treatment, treatment by protein precipitant (such as salt precipitation), centrifugation, cell lysis by osmosis, ultrasonic treatment, supercentrifugation, molecular sieve chromatography (gel filtration), adsorption chromatography, ion exchange chromatography, high performance liquid chromatography (HPLC), and any other liquid chromatography, and the combination thereof.

### Antibody-drug conjugate (ADC)

The present invention also provides an antibody-drug conjugate (ADC) based on the antibody according to the present invention.

Typically, the antibody-drug conjugate comprises the antibody and an effector molecule, wherein the antibody is conjugated to the effector molecule, and chemical conjugation is preferred. Preferably, the effector molecule is a therapeutically active drug. In addition, the effector molecule may be one or more of a toxic protein, a chemotherapeutic drug, a small-molecule drug or a radionuclide.

The antibody according to present invention and the effector molecule may be coupled by a coupling agent. Examples of the coupling agent may be any one or more of a non-selective coupling agent, a coupling agent utilizing a carboxyl group, a peptide chain, and a coupling agent utilizing a disulfide bond. The non-selective coupling agent refers to a compound that results in a linkage between an effector molecule and an antibody via a covalent bond, such as glutaraldehyde, etc. The coupling agent utilizing a carboxyl group may be any one or more of cis-aconitic anhydride coupling agents (such as cis-aconitic anhydride) and acyl hydrazone coupling agents (the coupling site is acyl hydrazone).

Certain residues on an antibody (such as Cys or Lys, etc.) are used to link a variety of functional groups, including imaging agents (such as chromophores and fluorophores), diagnostic agents (such as MRI contrast agents and radioisotopes), stabilizers (such as poly(ethylene glycol)) and therapeutic agents. An antibody can be conjugated to a functional agent to form a conjugate of the antibody-functional agent. A functional agent (e.g. a drug, a detection reagent, a stabilizer) is conjugated (covalently linked) to an antibody. A functional agent can be linked to an antibody either directly or indirectly via a linker.

Antibodies can be conjugated to drugs to form antibody-drug conjugates (ADCs). Typically, an ADC comprises a linker between a drug and an antibody. The linker can be a degradable or non-degradable linker. Typically, degradable linkers are easily degraded in an intracellular environment, for example, the linker is degraded at the target site, thereby releasing the drug from the antibody. Suitable degradable linkers include, for example, enzyme-degradable linkers, including peptidyl-containing linkers that can be degraded by protease (e.g. lysosomal protease or endosomal protease) in a cell, or sugar linkers, for example, glucuronide-containing linkers that can be degraded by glucuronidase. Peptidyl linkers may include, for example, dipeptides, such as valine-citrulline, phenylalanine-lysine or valine-alanine. Other suitable degradable linkers include, for example, pH sensitive linkers (e.g. linkers that are hydrolyzed at a pH of below 5.5, such as hydrazone linkers) and linkers that are degraded under reducing conditions (e.g. disulfide-bond linkers). A non-degradable linker typically releases a drug under conditions that the antibody is hydrolyzed by protease.

Prior to linkage to an antibody, a linker has a reactive group capable of reacting with certain amino acid residues, and the linkage is achieved by the reactive group. A thiol-specific reactive group is preferred, and includes, for example, a maleimide compound, a halogenated (e.g. iodo-, bromo- or chloro-substituted) amide; a halogenated (e.g. iodo-, bromo- or chloro-substituted) ester; a halogenated (e.g. iodo-, bromo- or chloro-substituted) methyl ketone, a benzyl halide (e.g. iodide, bromide or chloride); vinyl sulfone, pyridyl disulfide; a mercury derivative such as 3,6-di-(mercurymethyl)dioxane, wherein the counter ion is CH₃COO⁻, Cl⁻ or NO₃⁻; and polymethylene dimethyl sulfide thiosulfonate. The linker may include, for example, a maleimide linked to an antibody via thiosuccimide.

A drug may be any cytotoxic drug which inhibits cell growth or immunosuppression. In an embodiment, an antibody is linked to a drug via a linker, and the drug has a functional group that can form a bond with the linker. For example, a drug may have an amino group, a carboxyl group, a thiol group, a hydroxyl group, or a ketone group that can form a bond with a linker. When a drug is directly linked to a linker, the drug has a reactive group before being linked to an antibody.

Useful drugs include, for example, anti-tubulin drugs, DNA minor groove binding agents, DNA replication inhibitors, alkylating agents, antibiotics, folic acid antagonists, antimetabolites, chemotherapy sensitizers, topoisomerase inhibitors, vinca alkaloids, etc. Examples of particularly useful cytotoxic drugs include, for example, DNA minor groove binding agents, DNA alkylating agents, and tubulin inhibitors; typical cytotoxic drugs include, for example, auristatins, camptothecins, docamycin/duocarmycins, etoposides, maytansines and maytansinoids (e.g. DM1 and DM4), taxanes, benzodiazepines or benzodiazepine containing drugs (e.g. pyrrolo[1,4]benzodiazepines (PBDs), indolinobenzodiazepines and oxazolidinobenzodiazepines), and vinca alkaloids.

In the present invention, a drug-linker can be used to form an ADC in a simple step. In other embodiments, a bifunctional linker compound can be used to form an ADC in a two-step or multi-step process. For example, a cysteine residue is reacted with the reactive moiety of a linker in a first step, and then the functional group on the linker is reacted with a drug in the subsequent step, so as to form an ADC.

In general, the functional group on a linker is selected so that it can specifically react with the suitable reactive group on a drug moiety. As a non-limiting example, an azide-based moiety can be used to specifically react with the reactive alkynyl group on a drug moiety. The drug is covalently bound to the linker by 1,3-dipolar cycloaddition between the azide and alkynyl group. Other useful functional groups include, for example, ketones and aldehydes (suitable for reacting with hydrazides and alkoxyamines), phosphines (suitable for reacting with azides); isocyanates and isothiocyanates (suitable for reacting with amines and alcohols); and activated esters, for example, N-hydroxysuccinimide esters (suitable for reacting with amines and alcohols). These and other linkage strategies, for example, those described in "Bioconjugation Technology" (2nd Edition (Elsevier)), are well known to those skilled in the art. Those skilled in the art could understand that when a complementary pair of reactive functional groups are selected for a selective reaction between a drug moiety and a linker, each member of the complementary pair can be used for the linker, and can also be used for the drug.

The present invention further provides a method for preparing an ADC, which may further comprise: under conditions sufficient to form an antibody-drug conjugate (ADC), binding an antibody to a drug-linker compound.

In certain embodiments, the method according to the present invention comprises: under conditions sufficient to form an antibody-linker conjugate, binding an antibody to a bifunctional linker compound. In these embodiments, the method according to the present invention further comprises: under conditions sufficient to covalently link the drug moiety to the antibody via a linker, binding the antibody-linker conjugate to the drug moiety.

In some embodiments, an antibody-drug conjugate (ADC) has a formula as follows:

Ab(̵LU―D)ₚ

wherein:
Ab is an antibody,
LU is a linker;
D is a drug;
And the subscript p is a value selected from 1 to 8.

### Application

The present invention also provides use of the antibody, the antibody conjugate ADC, the recombinant protein, and/or immune cell of the present invention, for example for the preparation of diagnostic preparations or the preparation of drugs.

Preferably, the drug is for prevention and/or treatment of diseases associated with abnormal IL-5 expression or function.

In the present invention, the diseases associated with abnormal IL-5 expression or function are conventional diseases associated with abnormal IL-5 expression or function in the art. Preferably, the disease associated with abnormal IL-5 expression or function is asthma, atopic dermatitis, or inflammation diseases such as chronic obstructive pulmonary disease.

In the present invention, the asthma is a conventional asthma in the art, preferably eosinophilic asthma. In the invention, the specific dermatitis disease is a conventional specific dermatitis disease in the art, preferably an exogenous specific dermatitis or an endogenous specific dermatitis. In the invention, the chronic obstructive pulmonary disease is a conventional chronic obstructive pulmonary disease in the art, preferably chronic bronchitis.

Uses of the antibody, the ADC, the recombinant protein, and/or the immune cell of the present invention include (but are not limited to):
(i) diagnosis, prevention and/or treatment of occurrence and/or development of asthma, especially asthma with high IL-5 expression. The asthma includes (but is not limited to) eosinophilic asthma.
(ii) diagnosis, prevention and/or treatment of specific dermatitis including (but not limited to)exogenous specific dermatitis or endogenous specific dermatitis.
(iii) diagnosis, prevention and/or treatment of chronic obstructive pulmonary disease including (but not limited to) chronic bronchitis.

### Use for detection and kit

The antibody or ADC thereof of the present invention can be used for detection, for example, for detecting samples, thereby providing diagnostic information.

In the present invention, the samples (specimens) used include cells, tissue samples and biopsy specimens. The term "biopsy" used in the present invention shall include all kinds of biopsy known to those skilled in the art. Therefore, the biopsy used in the present invention may include, for example, body fluid samples of patients with asthma, tissue samples prepared by endoscopic methods or organ puncture or needle biopsy.

The samples used in the present invention include fixed or preserved cell or tissue samples.

The present invention also provides a kit comprising the antibody (or a fragment thereof) of the present invention. In a preferred embodiment of the present invention, the kit further includes a container, an instruction for use, buffer, and the like. In a preferred embodiment, the antibody of the present invention can be immobilized on a detection plate.

### Pharmaceutical composition

The invention further provides a composition. In the preferred examples, the composition is a pharmaceutical composition comprising the antibody, or an active fragment, a fusion protein or an ADC thereof, or a corresponding immune cell, and a pharmaceutically acceptable carrier. In general, these substances may be formulated in a non-toxic, inert and pharmaceutically acceptable aqueous carrier medium, wherein the pH is generally about 5-8, preferably, pH is about 6-8, though the pH value may be varied depending on the nature of the substances to be formulated and the condition to be treated.

The formulated pharmaceutical composition may be administered by conventional routes, including (but not limited to): intratumoral, intraperitoneal, intravenous, or topical administration. Typically, the administration route of the pharmaceutical composition of the present invention is preferably injection or oral administration. The injection administration preferably includes intravenous injection, intramuscular injection, intraperitoneal injection, intradermal injection, or subcutaneous injection. The pharmaceutical composition is in one of a variety of conventional dosage forms in the art, preferably in solid, semi-solid or liquid form, and can be an aqueous solution, a non-aqueous solution or a suspension, and more preferably tablets, capsules, granules, injection or infusion, etc.

The antibody of the present invention can also be used for cell therapy by expressing the nucleotide sequence in a cell, for example, the antibody is used for chimeric antigen receptor T cell immunotherapy (CAR-T) and the like.

The pharmaceutical composition of the present invention is a pharmaceutical composition for prevention and/or treatment of diseases associated with abnormal IL-5 expression or function.

The pharmaceutical composition according to the present invention can be directly used for binding to an IL-5 protein molecule, and thus can be used for preventing and treating diseases such as asthma.

The pharmaceutical composition according to the present invention comprises a safe and effective amount (e.g. 0.001-99 wt %, preferably 0.01-90 wt %, preferably 0.1-80 wt %) of the monoclonal antibody according to the present invention (or a conjugate thereof) and a pharmaceutically acceptable carrier or excipient. Such carriers include, but are not limited to, saline, buffers, glucose, water, glycerin, ethanol and a combination thereof. The pharmaceutical preparation should be matched to the method of administration. The pharmaceutical composition of the present invention can be prepared in the form of injection, for example, prepared by a conventional method using physiological saline or an aqueous solution containing glucose and other adjuvants. Pharmaceutical compositions such as injections and solutions are preferably prepared under sterile conditions. The dosage of active ingredient is therapeutically effective amount, for example from about 1 microgram per kilogram body weight to about 5 milligrams per kilogram body weight per day. Further, the polypeptide of the present invention can also be used in combination with the other therapeutic agents.

In the present invention, preferably, the pharmaceutical composition of the present invention further comprises one or more pharmaceutical carriers. The pharmaceutical carrier is a conventional pharmaceutical carrier in the art, and the pharmaceutical carrier can be any suitable physiologically or pharmaceutically acceptable pharmaceutical excipient. The pharmaceutical excipient is a conventional pharmaceutical excipient in the art, and preferably includes pharmaceutically acceptable excipients, fillers or diluents. More preferably, the pharmaceutical composition comprises 0.01-99.99% of the above-mentioned protein and 0.01-99.99% of the pharmaceutically acceptable carrier, wherein the percentage is the mass percentage of the pharmaceutical composition.

In the present invention, preferably, the administration amount of the pharmaceutical composition is an effective amount, and the effective amount is an amount that can alleviate or delay the progression of the disease, and the degenerative or traumatic condition. The effective amount can be determined on an individual basis and will be partly based on consideration of the symptoms to be treated and the results sought. Those skilled in the art can determine the effective amount by using the above-mentioned factors such as individual basis and using no more than conventional experiments.

When a pharmaceutical composition is used, a safe and effective amount of the immunoconjugate is administered to a mammal, wherein the safe and effective amount is usually at least about 10 micrograms per kilogram of body weight, and in most cases does not exceed about 50 mg/kg body weight, preferably the dose is about 10 µg /kg body weight to about 20 mg/kg body weight. Of course, the particular dose should also depend on various factors, such as the route of administration, patient healthy status, which are well within the skills of an experienced physician.

The present invention provides use of the above-mentioned pharmaceutical composition in the preparation of a medicine for preventing and/or treating diseases associated with abnormal IL-5 expression or function. Preferably, the disease associated with abnormal IL-5 expression or function is asthma, atopic dermatitis, or inflammation diseases such as chronic obstructive pulmonary disease.

### Method and composition for detecting IL-5 protein in sample

The present invention also provides a method for detecting IL-5 protein in a sample (for example, detecting cells over-expressing IL-5), which comprises the following steps: contacting the above-mentioned antibody with a sample to be tested *in vitro,* and detecting whether the above-mentioned antibody binds to the sample to be tested, to form an antigen-antibody complex.

The meaning of overexpression is conventional in the art, which refers to the overexpression of RNA or protein of IL-5 protein in the sample to be tested (due to increased transcription, post-transcriptional processing, translation, post-translational processing and protein degradation changes), and local overexpression and increased functional activity (such as in the case of increased enzymatic hydrolysis of the substrate) due to changes in protein transport mode (increased nuclear localization).

In the present invention, the detection method for detecting whether an antigen-antibody complex is formed is a conventional detection method in the art, preferably a flow cytometry (FACS) detection.

The present invention provides a composition for detecting IL-5 protein in a sample, which comprises the above-mentioned antibody, recombinant protein, antibody conjugate, immune cell, and a combination thereof as an active ingredient. Preferably, it also comprises a compound composed of the functional fragments of the above-mentioned antibody as an active ingredient.

On the basis of conforming to common knowledge in the art, the above-mentioned preferred conditions can be combined arbitrarily to obtain preferred embodiments of the present invention.

### The main advantages of the invention are:

According to the invention, a group of mouse-derived full-length antibodies combined with IL-5 are screened out using hybridoma technology. The variable regions of these antibodies can be sequenced for amino acids and DNA by conventional molecular biological methods. All variable regions contain three complementary determining regions or hypervariable regions, CDR1, CDR2 and CDR3. The murine antibody obtained from hybridoma can be combined with the constant region of human antibody to form mouse-human chimeric antibody. This group of antibodies or antigen binding fragments has a series of excellent characteristics, including high affinity and specificity for binding human IL-5 protein, effectively blocking the binding of IL-5 to its receptor, neutralizing the proliferation of CTLL-2 and TF-1 stimulated by IL-5, etc. Specific features include:
(1) having high affinity with human IL-5 protein, and its dissociation constant is usually less than 5×10⁻¹⁰;
(2) having strong binding ability with human and monkey (cynomolgus macaques) IL-5; some antibodies also have strong binding ability with mouse IL-5.
(3) can obviously block the binding of IL-5 to its receptor, which is verified by receptor ligand binding block assay;
(4) can obviously neutralize the activity of IL-5 and inhibit the proliferation of CTLL-2 cells in the neutralization assay of antibody on CTLL-2 proliferation stimulated by IL-5; wherein the antibody has obvious inhibitory effect at the concentration of 10⁻¹¹M.
(5) can obviously neutralize the activity of IL-5 and inhibit the proliferation of TF-1 cells in the neutralization assay of antibody on TF-1 proliferation stimulated by IL-5.

The invention is further illustrated by the following specific examples. It is to be understood that these examples are for illustrative purposes only and are not intended to limit the scope of the invention. The experimental methods without detailed conditions in the following examples are generally in accordance with the conditions described in the conventional conditions such as Sambrook. J et al. "Guide to Molecular Cloning Laboratory" (translated by Huang Peitang et al., Beijing: Science Press, 2002), or in accordance with the conditions recommended by the manufacturer (for example, product manuals). Percentages and parts are by weight unless otherwise stated. The experimental materials and reagents used in the following examples are commercially available unless otherwise specified.

The room temperature described in the examples is a conventional room temperature in the art, and is generally 10-30°C.

### EXAMPLE 1 Expression and purification of recombinant human IL-5

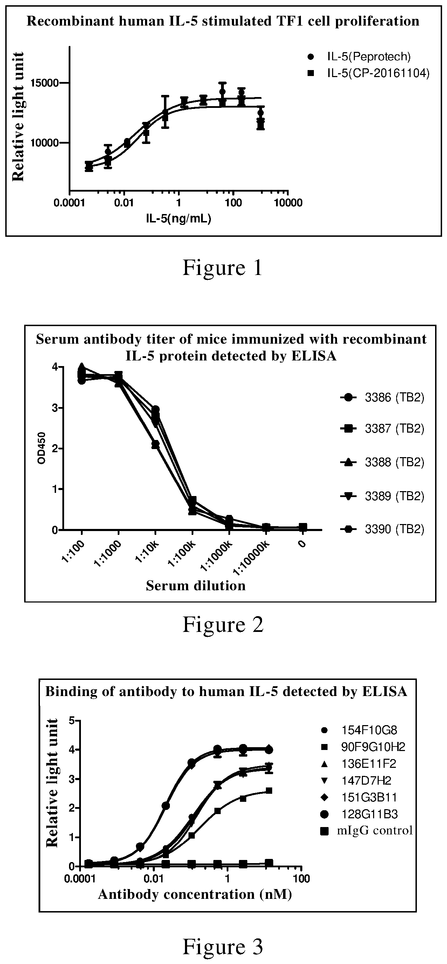

DNA encoding six histidines was added to the 3'end of the DNA fragment encoding Met1-Ser134 in the amino acid sequence of human IL-5 protein (NP_000870.1) by PCR, and the obtained DNA fragment encoding his-tagged recombinant human IL-5 protein was cloned into the expression vector by molecular biological methods. The plasmid was amplified by *Escherichia coli* and purified by alkaline lysis. The expression plasmid was transfected instantaneously and the recombinant protein was expressed by insect cell SF21. After 5-7 days of culture, centrifugal filtration was carried out to collect cell culture supernatant. The recombinant human IL-5 protein with His tag was purified by Ni-NTA affinity chromatography, and then further purified by molecular sieve column to remove impurities such as macromolecular polymers. The purified protein was stored in PBS buffer, filtered aseptically by 0.22 micron filter, then packed separately and stored at -80°C. The protein samples must undergo a series of quality control tests before use, including protein concentration, purity, molecular weight, biological activity, and so on. The biological activity of recombinant human IL-5 protein was detected by experiments. Part of the experimental results are shown in Table 1 and Figure 1.

**Table 1 Biological activity of recombinant human IL-5 protein was examined by TF-1 cell proliferation assay**

| Relative light unit | IL-5(Peprotech) | | IL-5(CP-20161104) | |
|---|---|---|---|---|
| IL-5 concentration (ng/mL) | | | | |
| 1000.000 | 11993.51 | 13032.22 | 11139.83 | 11753.8 |
| 200.000 | 14546.12 | 13873.27 | 13120.53 | 13692.45 |
| 40.000 | 13545.26 | 14987.68 | 13435.92 | 12973.34 |
| 8.000 | 13831.22 | 13646.19 | 13217.25 | 13915.33 |
| 1.600 | 13015.39 | 13784.96 | 13456.95 | 13709.27 |
| 0.320 | 11265.99 | 13890.1 | 12149.1 | 11934.63 |
| 0.064 | 11413.18 | 11320.66 | 11615.03 | 10008.61 |
| 0.013 | 9970.76 | 10315.59 | 10008.61 | 9642.746 |
| 0.003 | 8725.992 | 9802.548 | 8801.687 | 7922.78 |
| 0.001 | 7910.164 | 8410.594 | 8339.104 | 7670.462 |

Table 1 shows that the recombinant human IL-5 protein can stimulate the proliferation of TF-1 cells, and the biological activity of recombinant human IL-5 protein is basically consistent with that of commercial protein. Among them, IL-5 (Peprotech) refers to commercial IL-5 protein purchased from Peprotech as positive control; IL-5 (CP-20161104) is the above mentioned recombinant human IL-5 protein.

### EXAMPLE 2 Preparation of anti-human IL-5 antibody using hybridoma technique

### 2.1 Mice were immunized with recombinant human IL-5 protein

Balb/c and SJL/J mice aged 6-8 weeks (provided by Shanghai slake) were used for protein immunization. Mice were fed under SPF conditions after receiving. The primary immunization dose was 50 micrograms of recombinant human IL-5 protein per mouse. The protein was emulsified with Freund's complete adjuvant, and then injected subcutaneously into the tail with 0.25 ml. Two weeks after the primary immunization, booster immunization was preformed. Recombinant human IL-5 protein (25 microgram protein per mouse) was emulsified with Freund's incomplete adjuvant and then intraperitoneally injected with 0.25 ml. After that, the interval of each booster immunization was 3 weeks. Serum samples were collected one week after each booster immunization, and the antibody titer in serum was detected by ELISA and the antibody activity in serum was detected by receptor ligand binding block test. Mice with higher serum titer and better blocking on IL-5 binding to receptor would be preferentially selected for cell fusion and hybridoma cell preparation, and the remaining mice would continue to booster immunization for standby. Part of the experimental results are shown in Figure 2 and Table 2.

**Table 2 Detection of serum antibody titer of mice immunized with immunogen of recombinant human IL-5 protein by ELISA**

| OD₄₅₀ₙₘ | Batch | | | | |
|---|---|---|---|---|---|
| Serum dilution | 3386 (TB2) | 3387 (TB2) | 3388 (TB2) | 3389 (TB2) | 3390 (TB2) |
| 1:100 | 3.67 | 3.82 | 4 | 3.81 | 3.77 |
| 1:1000 | 3.77 | 3.71 | 3.6 | 3.8 | 3.69 |
| 1:10k | 2.96 | 2.79 | 2.09 | 2.6 | 2.12 |
| 1:100k | 0.72 | 0.73 | 0.46 | 0.59 | 0.5 |
| 1:1000k | 0.13 | 0.15 | 0.1 | 0.12 | 0.28 |
| 1: 10000k | 0.07 | 0.06 | 0.06 | 0.05 | 0.05 |
| Blank control | 0.06 | 0.06 | 0.06 | 0.07 | 0.06 |

Table 2 shows that the serum of mice immunized with recombinant human IL-5 protein has different degrees of binding to recombinant human IL-5 protein, showing antigen-antibody reaction. Among them, the highest dilution (i.e. dilution multiple) of serum was about 100,000. Among them, the blank control was 1% (w/w) BSA, and the batch TB2 referred to the mice serum on the seventh day after the second booster immunization. The data in the table is the value of OD450nm.

### 2.2 Preparation of hybridoma cells and screening of antibody

Usually, the titer of most mice can reach more than 1: 1000 after 2-3 immunizations, which can be used to collect lymphocytes for cell fusion and hybridoma preparation. Before cell fusion, mice were intraperitoneally injected with 50-100 micrograms of recombinant human IL-5 protein per mouse, for the last immunization. After 3-5 days, the mice were sacrificed and splenocytes were collected. NH₄OH was added to a final concentration of 1% to lyse the red blood cells in the spleen cell suspension. Cells were washed 2-3 times by centrifugation with DMEM basal medium, and then mixed with mouse myeloma cells SP2/0 at a ratio of 5: 1. Cell fusion was performed using traditional PEG cell fusion method or high-efficiency electrofusion method. The fused cells were diluted into DMEM selective medium containing 20% fetal bovine serum and 1 × HAT, and added to a 96-well cell culture plate at 1 × 10⁵/20 microliters per well, and placed in a 5% CO₂, 37°C incubator. After 10-14 days, the supernatant of cell fusion plate was screened by ELISA. The positive clones were amplified into a 24-well plate for expansion culture. After 2-3 days, the supernatant of 24-well plate was re-examined, including detection of the binding activity of antibody in the supernatant to human IL-5 protein by ELISA, detection of the blocking of antibody in the supernatant on the binding activity of human IL-5 to its receptor, and detection of neutralization of antibody in the supernatant against CTLL-2 proliferation experiment stimulated by IL-5, to test its biological activity with human IL-5.

According to the secondary screening results of 24-well plate samples, positive clones were selected for subcloning on a 96-well plate by limited dilution method. 7-10 days after subcloning, ELISA were used for preliminary screening, and 3-4 positive monoclones were selected and expanded to a 24-well plate for continue culture. After 2-3 days, the supernatant was re-examined according to the test methods between subclones, including ELISA, receptor ligand binding blocking test, neutralization of antibody in the supernatant against CTLL-2 proliferation test stimulated by IL-5. According to the results of 24-well plate samples, the best subclones were selected for expanded culture, liquid nitrogen cryopreservation, antibody production and purification.

### 2.3 Preliminary production and purification of hybridoma antibody

The hybridoma cells was expanded and domesticated with a production medium (Hybridoma serum free medium, Invitrogen) in the present invention, and then inoculated into a spinner flask for cell culture. 200-500 ml of production medium was added to each 2-liter culture spinner flask, and the inoculation cell density was 0.5-1.0 × 10⁵/ml. The inoculation cell density sometimes needs to be properly adjusted according to the growth state of cells. After inoculating cells aseptically, the spinner flask was placed at a spinner flask machine in a 37°C incubator. After 10-14 days of continuous rotating culture, cell culture solution was collected, cells was removed and the culture was filtered to clarify. The treated cell culture supernatant can be purified immediately or cryopreserved at -30°C.

Monoclonal antibodies in the supernatant of hybridoma cell culture can be purified by Protein G affinity chromatography column. According to the size of the sample,the chromatographic column was prepared with corresponding volume. For small volume purification of 200-300 ml, 1-2 ml protein G column was needed. The protein G column was first equilibrated with equilibration buffer (PBS, pH7.2), and then the culture supernatant was loaded onto the chromatography column. After loading, the chromatography column was washed with equilibration buffer to remove other unbound proteins. The antibody bound to the column was eluted with 0.1 M, pH 2.5 glycine hydrochloride buffer, and the eluted antibody (UV absorption peak) was collected. 10% volume of 1.0 M Tris-HCl buffer was added to neutralize pH and then dialysis with PBS was immediately performed. The dialyzed antibodies were filtered and sterilized with a 0.22 micron filter, and then aseptically packaged and stored. Small samples were taken for detection and analysis of protein concentration, purity, and internal toxicity. Endotoxin (LPS) is a component in the cell wall of gram-negative bacteria, which is a common impurity in the process of antibody purification, and has potential influence on many biological experiments *in vitro* and *in vivo.* Therefore, it is necessary to strictly control the contamination of endotoxin in the antibody purification process, and the concentration of endotoxin in the final product should be controlled within 1.0 EU/mg as much as possible. Part of the results are shown in Table 3.

**Table 3 Antibody detection and analysis**

| Clone number | Protein concentration (mg/ml) | Antibody purity | Endotoxin (EU/mg) |
|---|---|---|---|
| 69F2F3 | 0.65 | 100% | 0.74 |
| 102A2E11B6 | 0.52 | 100% | 1.74 |
| 100F4E11B3 | 0.15 | 100% | 6.31 |
| 90F9G10H2 | 0.59 | 100% | 1.06 |
| 136E11F2 | 0.63 | 100% | 0.18 |
| 147D7H2 | 0.1 | 100% | <1.202 |
| 151G3B11 | 0.93 | 100% | <0.134 |
| 154F10G8 | 0.41 | 100% | <0.305 |
| 128G11B3 | 0.74 | 100% | <0.168 |

### EXAMPLE 3 Detection of lead antibody

### 3.1 Antigen binding assay:

The titer of antibody reacting with human IL-5 protein and the cross reaction with mouse and monkey IL-5 protein were detected and analyzed by enzyme-linked immunosorbent assay (ELISA). Streptavidin was diluted with PBS to a final concentration of 1.0 µg/ml, and then added to a 96-well enzyme-labeled plate as 100 microliters per well. The plate was incubated at 4°C overnight. On the second day, the plate was washed twice with washing solution (PBS+0.01% Tween20). The blocking solution (PBS+0.05% Tween20 + 2% BSA) was added for blocking at 37°C for 1-2 hours. Then the blocking solution was discarded. The biotin-labeled human IL-5 was diluted with sample diluent (PBS+0.05% Tween20 + 0.2% BSA) to 0.5 µg/ml, and added to an enzyme-labeled plate at 50-100 microliters per well, and incubated at 37°C for 1 hour. The plate was washed with plate washing solution (PBS+0.05% Tween 20) for 2-3 times. 50-100 microliters of antibody samples to be tested were added to each well, and incubated at 37°C for 1 hour. Then the plate was washed with plate washing solution (PBS+0.05% Tween20) for 2-3 times. The horseradish peroxidase labeled secondary antibody against human or mouse IgG was added, and reacted at 37°C for 1 hour. The plate was washed with plate washing solution (PBS+0.05% Tween 20) for 2-3 times. 100 microliters of TMB substrate was added to each well. After incubation at room temperature for 15 minutes, 50 microliters of termination solution (1.0 N HCl) was added to each well. The value of O.D. 450nm was read by ELISA plate reader (SpectraMax M5e, Molecular Device). Part of the experimental results are shown in Figures 3-5 and Tables 4-6.

**Table 4 Detection of binding activity of lead antibody to human IL-5 by ELISA**

| OD₄₅₀ₙₘ | Clone number | | | | | | | | | | | | | |
|---|---|---|---|---|---|---|---|---|---|---|---|---|---|---|
| Antibody Concentration (nM) | 154F10G8 | | 90F9G10H2 | | 136E11F2 | | 147D7H2 | | 151G3B11 | | 128G11B3 | | mIgG control | |
| 13.3333 | 3.298 | 3.491 | 2.514 | 2.667 | 3.506 | 3.468 | 3.208 | 3.518 | 4.069 | 4.043 | 3.95 | 4.041 | 0.108 | 0.115 |
| 2.6667 | 3.226 | 3.168 | 2.303 | 2.34 | 3.355 | 3.124 | 3.087 | 3.199 | 4.068 | 3.803 | 4.017 | 4.085 | 0.079 | 0.079 |
| 0.5333 | 2.763 | 2.69 | 1.84 | 1.972 | 2.896 | 2.68 | 2.759 | 2.764 | 4.03 | 3.75 | 3.958 | 4.089 | 0.08 | 0.077 |
| 0.1067 | 1.694 | 1.705 | 1.046 | 1.085 | 1.558 | 1.602 | 1.42 | 1.473 | 3.503 | 3.399 | 3.538 | 3.579 | 0.08 | 0.079 |
| 0.0213 | 0.595 | 0.64 | 0.393 | 0.368 | 0.534 | 0.613 | 0.438 | 0.475 | 2.018 | 2.106 | 2.042 | 2.116 | 0.074 | 0.079 |
| 0.0043 | 0.205 | 0.193 | 0.14 | 0.137 | 0.187 | 0.175 | 0.158 | 0.159 | 0.625 | 0.609 | 0.666 | 0.711 | 0.078 | 0.072 |
| 0.0009 | 0.093 | 0.092 | 0.09 | 0.087 | 0.092 | 0.095 | 0.088 | 0.09 | 0.185 | 0.186 | 0.204 | 0.2 | 0.075 | 0.075 |
| 0.0002 | 0.079 | 0.07 | 0.081 | 0.076 | 0.075 | 0.07 | 0.073 | 0.074 | 0.103 | 0.094 | 0.106 | 0.103 | 0.076 | 0.075 |

**Table 5 Detection of binding activity of lead antibody to monkey IL-5 by ELISA**

| OD450nm | Clone number | | | | | | | | | | | | | |
|---|---|---|---|---|---|---|---|---|---|---|---|---|---|---|
| Antibody Concentration (nM) | 154F10G8 | | 90F9G10H2 | | 136E11F2 | | 147D7H2 | | 151G3B11 | | 128G11B3 | | mIgG control | |
| 13.3333 | 2.914 | 2.727 | 2.408 | 2.447 | 3.526 | 3.321 | 2.413 | 2.304 | 3.918 | 3.939 | 4.217 | 4.287 | 0.092 | 0.089 |
| 2.6667 | 2.565 | 2.547 | 2.184 | 2.318 | 3.126 | 3.101 | 2.118 | 2.023 | 3.923 | 3.882 | 4.229 | 4.218 | 0.073 | 0.072 |
| 0.5333 | 2.083 | 2.066 | 2.007 | 2.058 | 2.777 | 2.632 | 1.445 | 1.344 | 3.773 | 3.781 | 4.051 | 4.109 | 0.057 | 0.056 |
| 0.1067 | 1.181 | 1.053 | 1.127 | 1.15 | 1.425 | 1.38 | 0.644 | 0.516 | 3.047 | 3.27 | 3.453 | 3.442 | 0.056 | 0.056 |
| 0.0213 | 0.581 | 0.555 | 0.368 | 0.35 | 0.765 | 0.528 | 0.208 | 0.203 | 1.719 | 1.746 | 1.883 | 1.864 | 0.057 | 0.056 |
| 0.0043 | 0.202 | 0.165 | 0.15 | 0.139 | 0.236 | 0.192 | 0.095 | 0.088 | 0.574 | 0.598 | 0.71 | 0.645 | 0.055 | 0.055 |
| 0.0009 | 0.087 | 0.081 | 0.076 | 0.073 | 0.093 | 0.091 | 0.04 | 0.069 | 0.163 | 0.174 | 0.228 | 0.22 | 0.054 | 0.053 |
| 0.0002 | 0.079 | 0.068 | 0.063 | 0.061 | 0.067 | 0.079 | 0.075 | 0.074 | 0.11 | 0.125 | 0.114 | 0.109 | 0.055 | 0.056 |

**Table 6 Detection of binding activity of lead antibody to mouse IL-5 by ELISA**

| OD₄₅₀ₙₘ | Clone number | | | | | | | | | |
|---|---|---|---|---|---|---|---|---|---|---|
| Antibody Concentration (nM) | 69F2F3 | | 102A2E11B6 | | 100F4E11B3 | | 154F10G8 | | mIgG control | |
| 13.3333 | 2.821 | 2.809 | 1.809 | 1.988 | 0.587 | 0.551 | 4.006 | 4.039 | 0.052 | 0.054 |
| 2.6667 | 2.452 | 2.588 | 1.45 | 1.512 | 0.33 | 0.27 | 3.973 | 3.97 | 0.059 | 0.06 |
| 0.5333 | 1.824 | 1.71 | 1.025 | 1.193 | 0.189 | 0.176 | 3.706 | 3.632 | 0.053 | 0.052 |
| 0.1067 | 1.048 | 0.789 | 0.461 | 0.479 | 0.107 | 0.102 | 2.61 | 2.589 | 0.05 | 0.05 |
| 0.0213 | 0.265 | 0.256 | 0.139 | 0.138 | 0.065 | 0.063 | 1.114 | 1.134 | 0.05 | 0.332 |
| 0.0043 | 0.244 | 0.086 | 0.068 | 0.067 | 0.05 | 0.05 | 0.412 | 0.392 | 0.046 | 0.048 |
| 0.0009 | 0.059 | 0.058 | 0.051 | 0.052 | 0.05 | 0.049 | 0.205 | 0.216 | 0.049 | 0.049 |
| 0.0002 | 0.049 | 0.049 | 0.046 | 0.047 | 0.048 | 0.048 | 0.17 | 0.152 | 0.046 | 0.048 |

Tables 4-6 show that the lead antibodies bind to recombinant human IL-5 protein and recombinant monkey IL-5 at ELISA level. Some lead antibodies bind to mouse IL-5 at ELISA level. Wherein, the mIgG control is mouse IgG, and the data in the table are OD₄₅₀ₙₘ values.

### 3.2 Biological function analysis

### 3.2.1 Construction of stably expressed cell lines:

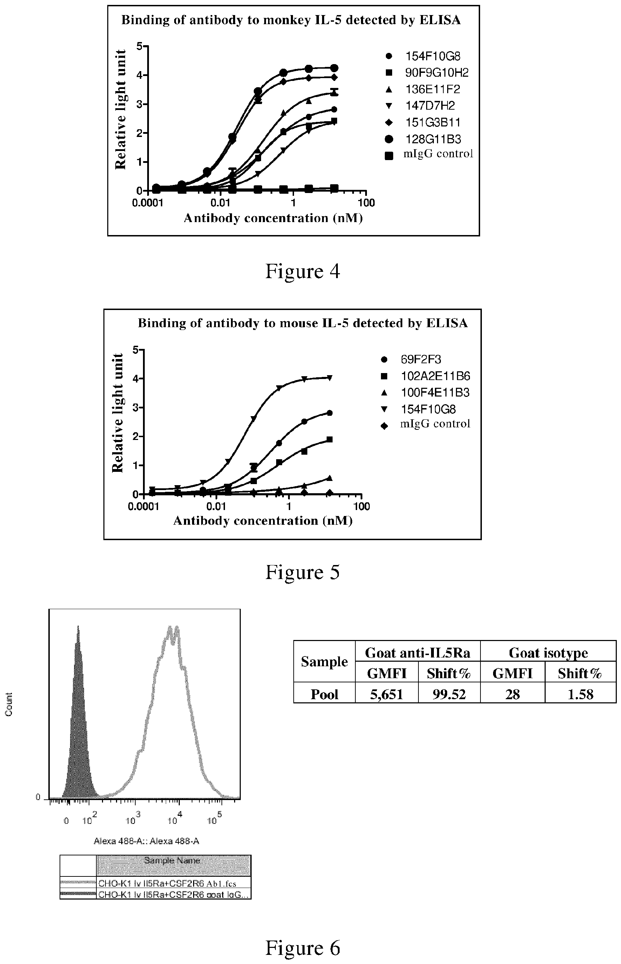

The full-length sequences of human IL-5Ra and CSF2RB chains were cloned into pIRES expression vector and packaged into lentivirus (Shanghai Jima). CHOK1 cells were infected with lentivirus containing genes expressing human IL-5Ra and CSF2RB chains, and then cultured in culture medium containing screening antibiotics. After 2 weeks, the infected cells were subcloned into a 96-well culture plate by limited dilution method. After the clones grew up, cells of the monoclonal well were expanded into 6-well plates or culture flasks. The receptor expression level of the expanded clones and their binding ability to ligand IL-5 protein were detected by flow cytometry with specific antibodies against each receptor. Monoclonal cell lines with better growth, higher expression level and strong binding were selected for further expanding culture and cryopreserved in liquid nitrogen. CTLL-2 cells were infected with virus particles expressing human IL-5Ra and CSF2RB chains, and monoclonal cell lines with better growth, higher expression level and strong binding were selected with the same method for expanding culture and preserved in liquid nitrogen. Some results are shown in Figure 6 and Table 7.

**Table 7 Expression level of IL-5Ra protein in CHOK1 cell line overexpressing full-length human IL-5Ra detected by flow cytometry**

| Sample | IL-5Ra antibody | | IgG subtype control | |
|---|---|---|---|---|
| | Mean fluorescence intensity | Positive cells (%) | Mean fluorescence intensity | Positive cells (%) |
| Transfected mother cells | 5,651 | 99.52 | 28 | 1.58 |

The results in Table 7 show that CHOK1 cells express IL-5Ra receptor, and the expression level of receptor on the cell surface is high, which can be used for follow-up test.

### 3.2.2 Receptor ligand binding block assay using flow cytometry:

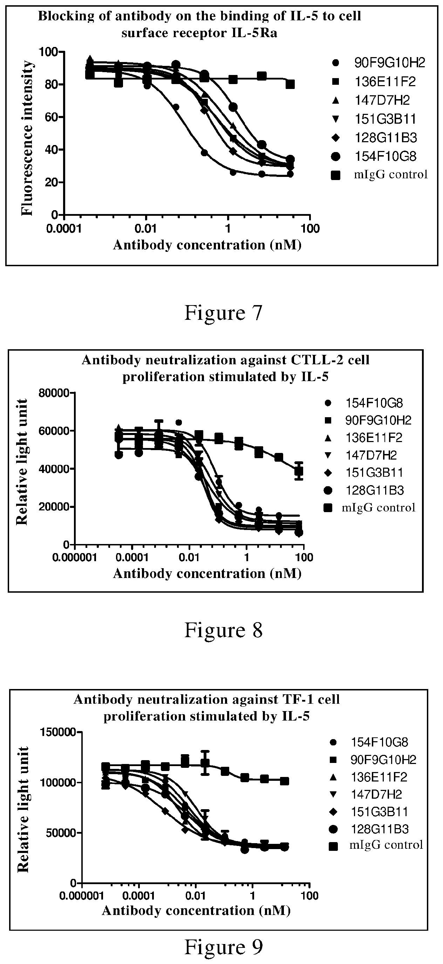

CHOK1 cell line overexpressing full-length human IL-5Ra/CSF2RB was expanded to a confluence of 75-90% in a T-175 cell culture flask, and the culture medium was discarded. The cells were washed with PBS for 1-2 times, and then digested with recombinant enzyme cell dissociation solution (TrypLE: Life technology). The cells were collected and washed with PBS buffer solution for 1-2 times. Then the cells were counted and diluted to 1-2×10⁶ cells per milliliter with blocking solution (PBS, 2% fetal bovine serum), incubated on ice for 20-30 minutes, and then washed twice with a blocking solution (PBS, 2% fetal bovine serum). The collected cells were suspended with a blocking solution (PBS, 2% fetal bovine serum) to 1 × 10⁶ cells/ml, and added to a 96-well FACS reaction plate (1 × 10⁵ cells per well) as 100 microliters per well. The gradient diluted antibody sample or culture supernatant was mixed with biotin labeled IL-5 (the final concentration is 30 ng/ml for hIL-5Ra1/hIL-4Ra; 20 ng/ml for HIL-5Ra2) and then was added to the cells as 100 microliters per well and incubated on ice for 1-2 hours. The plate was washed twice with blocking solution (PBS, 2% fetal calf serum) by centrifugation, added with 100 microliters of streptavidin labeled with fluorescence (Alexa 488) per well, and incubated on ice for 0.5-1.0 hours. The plate was washed 2-3 times by centrifugation with blocking solution (PBS, 2% fetal calf serum), added with 100 microliters of PBS per well to suspend cells, and detected using FACS (FACS Verse, BD) and the results were analyzed. Some results are shown in Figure 7 and Table 8.

**Table 8 Blocking of lead antibody on the binding of IL-5 to cell surface receptor IL-5Ra detected by FACS**

| Fluorescence intensity | Clone number | | | | | | |
|---|---|---|---|---|---|---|---|
| Antibody concentration (nM) | 90F9G10H2 | 136E11F2 | 147D7H2 | 151G3B11 | 128G11B3 | 154F10G8 | mIgG control |
| 33.3333 | 25 | 31 | 31 | 30 | 29 | 34 | 80 |
| 6.6667 | 25 | 36 | 36 | 34 | 32 | 43 | 85 |
| 1.3333 | 26 | 47 | 55 | 46 | 39 | 66 | 83 |
| 0.2667 | 38 | 69 | 74 | 69 | 64 | 86 | 84 |
| 0.0533 | 66 | 83 | 90 | 84 | 86 | 92 | 83 |
| 0.0107 | 79 | 84 | 88 | 87 | 87 | 91 | 83 |
| 0.0021 | 85 | 87 | 93 | 86 | 89 | 91 | 81 |
| 0.0004 | 92 | 93 | 96 | 93 | 92 | 90 | 86 |

Table 8 shows that the binding of IL-5 antibody to human IL-5 can block the binding of human IL-5 to the cell surface receptor IL-5Ra, wherein the mIgG control is mouse IgG, and the data in the table is average fluorescence intensity.

### 3.2.3 Antibody neutralization against CTLL-2 proliferation experiment stimulated by IL-5.

CTLL-2 cells (ATCC) overexpressing full-length human IL-5Ra/CSF2RB were cultured in 1640 medium containing 10% fetal bovine serum (Gibco) (RPMI1640+10% FBS+2mM L-Glutamine + ImM Sodium Pyruvate+10ng/ml IL2 +1.5 ug/ml puromycin + 200ug/ml Hygromycin). When the cells were expanded to 75-90% confluence in a T-175 cell culture flask, the culture medium was discarded by centrifugation and the cells were collected. The collected cells were re-suspended in 1640 medium (Gibco) (RPMI1640 + 2% FBS + 2mM L-Glutamine + ImM Sodium Pyruvate) containing 2% fetal bovine serum. After counting, the cells were diluted to 2 × 10⁵ cells per milliliter and added to a 96-well cell culture plate as 50 microliters per well (1 × 10⁴ cells per well). The culture plate was placed in a 37°C, 5% CO₂ incubator. The gradient diluted antibody or culture supernatant was mixed with IL-5 (final concentration was 5 ng/ml), then added to the cell culture plate and cultured in a 37°C, 5% CO₂ incubator. After 48 hours, 50 microliters of Cell Titer-Glo reagent (Promega) was added to each well of the cell culture plate, and the relative luminescence intensity was detected by microplate reader. Part of the results are shown in Figure 8 and Table 9.

**Table 9 Neutralization of lead antibody against CTLL-2 cell proliferation stimulated by IL-5**

| RLU | Clone number | | | | | | | | | | | | | |
|---|---|---|---|---|---|---|---|---|---|---|---|---|---|---|
| Ab conc. (nM) | 154F10G8 | | 90F9G10H2 | | 136E11F2 | | 147D7H2 | | 151G3B11 | | 128G11B3 | | mIgG control | |
| 66.66666 | 11496.64 | 10928.79 | 7665.881 | 9552.866 | 6216.864 | 6365.508 | 10743.41 | 11275.3 | 6497.037 | 5235.433 | 6647.966 | 6312.923 | 43168.49 | 34361.45 |
| 13.33333 | 16170.42 | 14728.97 | 10369.69 | 12012.06 | 7580.901 | 9080.469 | 12936.93 | 13482.01 | 7411.369 | 6598.14 | 8168.888 | 8909.51 | 45781.71 | 46324.49 |
| 2.666667 | 19725.99 | 17122.65 | 13418.57 | 16602.86 | 10623.75 | 10247.77 | 13653.45 | 14449.09 | 8721.326 | 8840.014 | 11462.01 | 10060.12 | 51170.11 | 46188.8 |
| 0.5333334 | 21525.62 | 20158.43 | 16240.31 | 16170.42 | 13325.6 | 13198.81 | 16726.13 | 15191.99 | 12506.14 | 11719.28 | 15694.14 | 14058.6 | 56199.58 | 50465.38 |
| 0.1066667 | 36119.19 | 30003.95 | 18843.65 | 21468.84 | 15664.57 | 15074.36 | 26537.62 | 26309.04 | 13956.76 | 12211.61 | 16862.38 | 16064.45 | 58199.99 | 53498.81 |
| 0.02133333 | 56867.3 | 52272.14 | 44130.14 | 41946.13 | 45411.09 | 41764.91 | 49039.88 | 45162.76 | 39325.09 | 38823.97 | 36475.65 | 35355.89 | 59780.18 | 55928.2 |
| 0.004266667 | 64244.89 | 64349.72 | 47790.54 | 46563.13 | 56192.23 | 59051.46 | 50591.61 | 55932.54 | 53589.56 | 50916.89 | 50975.09 | 52002.27 | 57862.94 | 48241.73 |
| 0.000853333 | 64755.95 | 54526.04 | 55535.05 | 56011.16 | 65338.27 | 54745.13 | 56402.89 | 57290.85 | 56504 | 56293 | 51455.61 | 55295.39 | 58589.57 | 53792.09 |
| 0.000170667 | 57924.36 | 56343.13 | 54578.45 | 51429.11 | 55383.42 | 61482.25 | 56772.14 | 61031.7 | 58029.35 | 54684.12 | 47906.8 | 48845.8 | 57433.97 | 51016.91 |
| 3.41333E-05 | 58714.97 | 61274.63 | 58688.76 | 58435.42 | 61753.3 | 61871.34 | 58943.68 | 59783.28 | 60640.47 | 48587.11 | 46429.96 | 48127.22 | 58602.7 | 55004.59 |

Table 9 shows that by binding to human IL-5, the lead antibody can neutralize proliferation of CTLL-2 cells stimulated by IL-5. The data in Table 9 are the relative light unit values of ATP measured in the cell culture plate, wherein the mIgG control is mouse IgG.

### 3.2.4 Antibody neutralization against TF-1 proliferation experiment stimulated by IL-5.

TF-1 cells (ATCC) expressing endogenous human IL-5Ra/CSF2RB were cultured in 1640 medium (Gibco) containing 10% fetal bovine serum (RPMI1640 + 10% FBS). When the cells were expanded to 75-90% confluence in a T-175 cell culture flask, the culture medium was discarded by centrifugation and the cells were collected. The cells were washed with PBS for 3 times. The collected cells were re-suspended in 1640 medium (Gibco) (RPMI1640 + 2% FBS) containing 2% fetal bovine serum. After counting, the cells were diluted to 2 × 10⁵ cells per milliliter and added to a 96-well cell culture plate as 50 microliters per well (1 × 10⁴ cells per well). The culture plate was placed in a 37°C, 5% CO₂ incubator. The gradient diluted antibody or culture supernatant was mixed with IL-5 (final concentration was 5 ng/ml), then added to the cell culture plate and cultured in a 37°C, 5% CO₂ incubator. After 48 hours, 50 microliters of Cell Titer-Glo reagent (Promega) was added to each well of the cell culture plate, and the relative luminescence intensity was detected by microplate reader. Part of the results are shown in Figure 9 and Table 10.

**Table 10 Neutralization of lead antibody against TF-1 cell proliferation stimulated by IL-5**

| RLU | Clone number | | | | | | | | | | | | | |
|---|---|---|---|---|---|---|---|---|---|---|---|---|---|---|
| Ab conc. (nM) | 154F10G8 | | 90F9G10H2 | | 136E11F2 | | 147D7H2 | | 151G3B11 | | 128G11B3 | | mIgG control | |
| 13.33333 | 33824.62 | 35341.05 | 34596.45 | 37960.76 | 35048.18 | 37362 | 35483.73 | 37477 | 35338.44 | 36564.37 | 39002.46 | 33243.16 | 100840.8 | 101667.4 |
| 2.66667 | 34492.03 | 41969.77 | 34936.97 | 41043.56 | 36121 | 34129.93 | 39860.75 | 38103.59 | 37894.72 | 34280.51 | 36513.43 | 34810.16 | 101199.6 | 105936.7 |
| 0.53333 | 40625.86 | 42342.07 | 38087.88 | 39722.36 | 37034.7 | 39771.28 | 36173.89 | 39406.7 | 35138.66 | 37749.43 | 35259.82 | 31430.89 | 103434.2 | 106463.5 |
| 0.10667 | 42142.3 | 51540.55 | 43558.84 | 44344.3 | 42626.05 | 40412.24 | 42317.14 | 43633.87 | 40401.06 | 40437.38 | 44184.92 | 41218.98 | 108035 | 114497.9 |
| 0.02133 | 54782.26 | 57451.91 | 52398.64 | 54764.1 | 53690.55 | 43603.39 | 72166.16 | 55893.13 | 49854.31 | 47207.22 | 55708.07 | 54854.17 | 108062.2 | 130830.1 |
| 0.00427 | 76094.04 | 79662.66 | 73637.78 | 75903.35 | 61918.46 | 60872.93 | 90250.83 | 86341.48 | 51102.94 | 54685.36 | 68502.98 | 65087.38 | 116092.1 | 126837.9 |
| 0.00085 | 99335.41 | 103299 | 93074.46 | 97178.8 | 95507.44 | 89957.01 | 104857.5 | 105370.6 | 72729.17 | 70704.13 | 89441.77 | 87552.28 | 114520.6 | 117368.3 |
| 0.00017 | 114127.4 | 108275.1 | 101723.6 | 102708.8 | 105544.6 | 101853.4 | 116058.8 | 111718.1 | 86473.16 | 91331.45 | 93161.7 | 96313.87 | 112204.3 | 120642.9 |
| 0.00003 | 110018.5 | 114540.6 | 107112.8 | 109900.5 | 108035.7 | 106121.9 | 111100.6 | 112748.8 | 95354.31 | 98251.13 | 98153.4 | 101918.7 | 112208.9 | 116055.7 |
| 0.00001 | 108275.1 | 112869.8 | 109110.5 | 114817.5 | 108172.1 | 112204.2 | 110900.9 | 111550.1 | 103599.8 | 105465.9 | 94288.13 | 101260.1 | 113362.5 | 117922.4 |

Table 10 shows that the lead antibody can neutralize proliferation of TF-1 cells stimulated by IL-5 by binding to human IL-5. The data in Table 10 are the relative light unit values of ATP measured in the cell culture plate, wherein the mIgG control is mouse IgG.

### Example 4 Determination of amino acid sequences of light and heavy chain variable regions

Total RNA extraction: The hybridoma cell lines corresponding to the selected lead antibodies were resuscitated and cultured, and 1-5 × 10⁷ cells were collected by centrifugation. 1 ml Trizol was added to the cell precipitation, blew and sucked repeatedly to lyse the cells. The cell lysate was transfered to a 1.5 ml centrifuge tube, and placed at room temperature for 5 minutes. 0.2ml of chloroform was added, shaked for 15 seconds, placed for 2 minutes, and then centrifuged at 12000g at 4°C for 10 minutes. The supernatant aqueous liquid was transferred to a new 1.5mL centrifuge tube. 0.5 mL of isopropanol was added, mixed well, and placed at room temperature for 10 minutes, and then centrifuged at 12000g at 4°C for 15 minutes. The precipitate was collected, washed gently with 1 ml of 75% ethanol. After centrifugation, the supernatant was carefully aspirated, and the precipitate was dried. A proper amount of DEPC-treated H₂O was used to dissolve (55°C water bath to promote dissolution for 10 minutes) the RNA precipitate. The RNA concentration was determined by light absorption method.

Reverse transcription and PCR: 1µg of total RNA was taken, and a 20 µ 1 reaction system was prepared by adding dNTP, buffer and reverse transcriptase, etc, and reacted at 42°C for 60 minutes, and then reacted at 70°C for 10 minutes to inactivate the reverse transcriptase. 1 microliter of reverse transcriptate product (cDNA) was taken, and a 50 microliter PCR system was prepared by adding 25 pmol of primer, 1 microliter of DNA polymerase, matching buffer system and 250 µmol dNTPs. PCR program was set, comprising pre-denaturation at 95 °C for 3 minutes, denaturation at 95 °C for 30 seconds, annealing at 55 °C for 30 seconds, extension at 72 °C for 35 seconds, and further extension at 72 °C for 5 min after 35 cycles.

Cloning and sequencing: 5µl of PCR product was taken for agarose gel electrophoresis detection. Column recovery kit was used to purify the positive samples. Ligation reaction were preformed at 16°C for half an hour in a 10µl reaction system containing sample 50ng, T vector 50ng, ligase 0.5µl, and buffer 1µl. 5µl of the ligation product was taken and added to 100µl of competent cells. Ice bathed for 5 minutes, then heat shock in a 42°C water bath was performed for 1 minute, and cells were put back on ice for 1 minute, and added with 650µl antibiotic-free SOC medium. The cells were resuscitated on a shaker at 37°C at 200 RPM for 30 minutes. Then 200µl of the culture was taken out, spreaded on LB solid medium containing antibiotics and incubated overnight at 37°C in an incubator. On the next day, primers M13F and M13R on the T vector were used to configure a 30µl PCR system to perform a colony PCR. A pipette tip was used to dip the colony into the PCR reaction system and pipette, and 0.5µl was aspirated onto another LB solid petri dish containing antibiotics to preserve the strain. After the PCR reaction was over, 5µl of the reaction solution was take out for agar glycogel electrophoresis detection, and the positive samples were sequenced.

The sequencing results are shown in the sequence information of the present invention in the appendix.

### EXAMPLE 5 Antibody affinity test

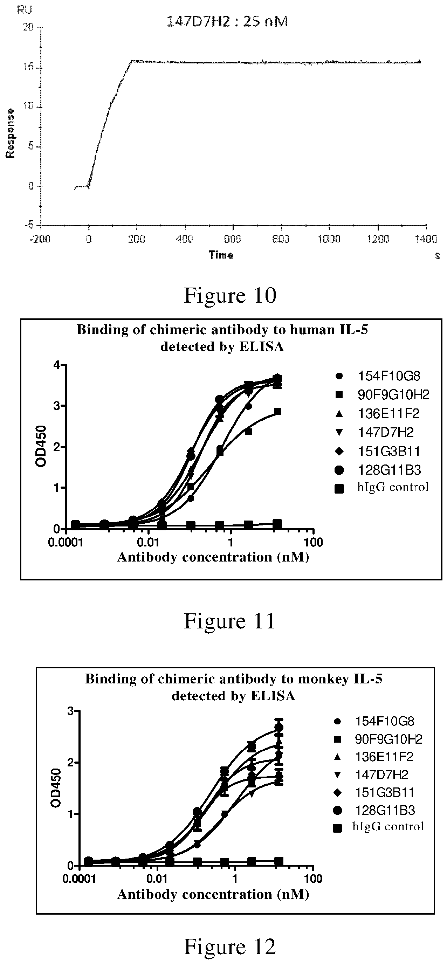

The human antigen IL-5 was immobilized on the surface of CM5 chip to 150-200 RU and the process was as follows: a freshly prepared mixture of 50 mM NHS and 200 mM EDC (1: 1) was used to activate for 200 sec, the antigen IL-5 was diluted to 1ug/ml with 10 mM NaOAc (pH 5.0), and captured on the chip at a flow rate of 10ul/min for about 1min. The remaining activated sites were blocked with 1M ethanolamine. Then the antibody to be tested was diluted to different concentrations (0, 12.5, 25nM), and then flowed through the chip surface at a flow rate of 30ul/min, respectively. The binding time was 180s and the dissociation time was 1200s. At the end of each cycle, the chip surface was regenerated with 10mM Glycine at pH 1.5. The kinetic rate constant needed to be subtracted the blank control, and the data was fitted with the global fit analysis method 1:1 combination model. The dissociation equilibrium rate constant (KD) was calculated according to the following formula: KD=kd/ka. Part of the results are shown in Table 11 and Figure 10.

**Table 11 Detection results of affinity between antibodies and human IL-5**

| Antibody | Ka (1/Ms) | Kd (1/s) | KD (M) |
|---|---|---|---|
| 69F2F3 | 3.678E+05 | 8.143E-06 | 2.214E-11 |
| 102A2E11B6 | 3.691E+05 | 2.324E-05 | 6.297E-11 |
| 100F4E11B3 | 1.259E+05 | 1.237E-04 | 9.824E-10 |
| 144D3C4A5 | 4.262E+05 | 1.429E-05 | 3.35E-11 |
| 90F9G10H2 | 1.601E+05 | < 1 E-05 | < 1/1.601 E-10 |
| 136E11F2 | 3.655E+05 | < 1 E-05 | < 1/3.655 E-10 |
| 147D7H2 | 1.985E+05 | < 1 E-05 | < 1/1.985 E-10 |
| 151G3B11 | 4.864E+05 | 9.037E-06 | 1.858E-11 |
| 128G11B3 | 2.869E+05 | < 1 E-05 | < 1/2.869 E-10 |
| 154F10G8 | 4.221E+05 | 6.650E-05 | 1.576E-10 |

Table 11 shows that the lead antibody has strong affinity with human IL-5.

### Example 6 Construction of mouse-human chimeric antibody, production and purification of the antibody

Plasmid construction and preparation: The hybridoma antibody heavy chain variable region sequence was cloned into the pCP expression vector containing the signal peptide and human heavy chain antibody IgG4 constant region, and the light chain variable region was recombined to the pCP vector containing the signal peptide and human antibody light chain kappa constant region, and the plasmid was verified by sequencing. Plasmids were extracted using alkaline lysis kit to increase the purity, filtered through a 0.22µm filter membrane for transfection.

Cell transfection: Freestyle 293F cells were used, and the medium was Freestyle 293 expression medium, added with 10% F68 to a final concentration of 0.1% when used. During transfection, the cell density was cultured to 1-1.5 × 10⁶ cells per milliliter; and the shaker was set to 37°C, 130RPM, with a CO₂ concentration of 8%. 5 ml of medium was taken and mixed well with PEI (200µg/ml). 5 ml of medium was taken and mixed well with a certain amount of plasmids (the amount of plasmids was 100µg/ml). After 5 minutes, the two solutions were combined and mixed well, let stand for 15 minutes. The mixture was added slowly into the cells, being shaken while added, to avoid excessive concentration of PEI. And the mixed solution was cultured in a shaker. On the next day, it was added with peptone (sigma) to a final concentration of 0.5%. On the 5-7 day, the antibody titer of the culture medium was tested. On the 6-7 day, it was centrifuged (3500RPM, 30min) and filtered to collect the supernatant for purification.

Antibody purification: for continuously used endotoxin-free chromatography columns and Protein A fillers, 0.1M NaOH was used for treatment for 30 minutes, or 5 column volumes of 0.5M NaOH was used for washing. For long-term unused column materials and chromatography columns, at least 1M NaOH was used for soaking for 1 hour, and non-endotoxic water was used for rinsing to neutrality, and the column material was washed with 10 column volumes of 1% Triton X100. 5 column volumes of PBS were used for equilibrate, and the filtered cell supernatant was loaded on the column, and the flow-through was collectd if necessary. After the samples were loaded, the column was washed with 5 column volumes of PBS. Elution was carried out with 5 column volumes of 0.1M Glycine-HCl with pH3.0, and the eluate was collected, and neutralized with 1/10 volume of 1M Tris-HCl (1.5M NaCl) with pH8.5. After the antibodies were harvested, they were dialyzed overnight in 1 × PBS to avoid endotoxin contamination. After dialysis, spectrophotometry or a kit was used to determine the concentration, and HPLC-SEC was used to determine the purity of the antibody, and an endotoxin detection kit was used to detect the content of antibody endotoxin. Part of the results are shown in Table 12.

**Table 12 Detection and analysis of chimeric antibody**

| Clone number | Protein concentration (mg/ml) | Antibody purity | Endotoxin (EU/mg) |
|---|---|---|---|
| 69F2F3 | 0.573 | > 90% | 1.71 |
| 102A2E11B6 | 1.193 | > 90% | 1.49 |
| 100F4E11B3 | 1.317 | > 90% | 1.03 |
| 144D3C4A5 | 0.67 | > 90% | 0.22 |
| 90F9G10H2 | 1.101 | > 90% | 0.75 |
| 136E11F2 | 1.156 | > 90% | 0.74 |
| 147D7H2 | 1.073 | > 90% | 0.63 |
| 151G3B11 | 0.721 | > 90% | 0.96 |
| 128G11B3 | 1.149 | > 90% | 0.53 |
| 154F10G8 | 0.51 | > 90% | 1.96 |

### EXAMPLE 7 Assay of mouse-human chimeric antibody

### 7.1 Antigen binding assay

The titer of chimeric antibody reacting with human IL-5 protein and the cross reaction with mouse and monkey IL-5 protein were detected and analyzed by enzyme-linked immunosorbent assay (ELISA). The experimental process was the same as that of Example 4.1, and see 3.1 for details. Part of the experimental results are shown in Figures 11-13 and Tables 13-15.

**Table 13 Detection of binding activity of chimeric antibody to human IL-5 by ELISA**

| OD₄₅₀ₙₘ | Clone number | | | | | | | | | | | | | |
|---|---|---|---|---|---|---|---|---|---|---|---|---|---|---|
| Ab conc. (nM) | 154F10G8 | | 90F9G10H2 | | 136E11F2 | | 147D7H2 | | 151G3B11 | | 128G11B3 | | hIgG control | |
| 13.3333 | 3.701 | 3.707 | 2.821 | 2.903 | 3.795 | 3.662 | 3.44 | 3.718 | 3.704 | 3.525 | 3.621 | 3.655 | 0.172 | 0.07 |
| 2.6667 | 2.959 | 3.011 | 2.365 | 2.375 | 3.353 | 3.383 | 3.221 | 3.347 | 3.468 | 3.503 | 3.359 | 3.579 | 0.09 | 0.088 |
| 0.5333 | 1.933 | 1.999 | 1.902 | 1.812 | 2.673 | 2.714 | 2.741 | 2.909 | 3.032 | 2.928 | 3.11 | 3.201 | 0.079 | 0.078 |
| 0.1067 | 0.716 | 0.77 | 1.056 | 1.002 | 1.454 | 1.473 | 1.255 | 1.3 | 1.872 | 1.919 | 1.703 | 1.845 | 0.078 | 0.078 |
| 0.0213 | 0.224 | 0.221 | 0.395 | 0.402 | 0.467 | 0.507 | 0.406 | 0.385 | 0.622 | 0.678 | 0.622 | 0.603 | 0.079 | 0.076 |
| 0.0043 | 0.108 | 0.106 | 0.168 | 0.173 | 0.17 | 0.171 | 0.172 | 0.156 | 0.194 | 0.244 | 0.204 | 0.211 | 0.074 | 0.077 |
| 0.0009 | 0.081 | 0.087 | 0.128 | 0.134 | 0.108 | 0.115 | 0.102 | 0.104 | 0.127 | 0.114 | 0.118 | 0.112 | 0.08 | 0.078 |
| 0.0002 | 0.083 | 0.098 | 0.123 | 0.135 | 0.108 | 0.085 | 0.103 | 0.101 | 0.098 | 0.127 | 0.096 | 0.106 | 0.082 | 0.08 |

**Table 14 Detection of binding activity of lead antibody to monkey IL-5 by ELISA**

| OD₄₅₀ₙₘ | Clone number | | | | | | | | | | | | | |
|---|---|---|---|---|---|---|---|---|---|---|---|---|---|---|
| Ab conc. (nM) | 154F10G8 | | 90F9G10H2 | | 136E11F2 | | 147D7H2 | | 151G3B11 | | 128G11B3 | | hIgG control | |
| 13.3333 | 2.301 | 1.965 | 1.873 | 1.645 | 2.543 | 2.297 | 1.576 | 1.735 | 2.152 | 2.208 | 2.522 | 2.839 | 0.092 | 0.092 |
| 2.6667 | 1.606 | 1.608 | 1.547 | 1.711 | 2.061 | 1.888 | 1.435 | 1.344 | 1.876 | 1.677 | 2.398 | 2.221 | 0.082 | 0.075 |
| 0.5333 | 0.988 | 0.997 | 1.661 | 1.364 | 1.678 | 1.623 | 1.031 | 0.983 | 1.533 | 1.815 | 1.769 | 1.9 | 0.065 | 0.063 |
| 0.1067 | 0.399 | 0.409 | 1.013 | 0.677 | 0.862 | 0.781 | 0.405 | 0.448 | 0.947 | 0.701 | 1.068 | 1.036 | 0.068 | 0.073 |
| 0.0213 | 0.154 | 0.157 | 0.415 | 0.233 | 0.309 | 0.308 | 0.168 | 0.173 | 0.291 | 0.3 | 0.404 | 0.386 | 0.072 | 0.062 |
| 0.0043 | 0.096 | 0.092 | 0.163 | 0.12 | 0.203 | 0.173 | 0.092 | 0.092 | 0.146 | 0.16 | 0.167 | 0.16 | 0.066 | 0.064 |
| 0.0009 | 0.071 | 0.067 | 0.096 | 0.081 | 0.089 | 0.098 | 0.071 | 0.082 | 0.095 | 0.128 | 0.095 | 0.09 | 0.063 | 0.064 |
| 0.0002 | 0.073 | 0.072 | 0.073 | 0.074 | 0.092 | 0.093 | 0.093 | 0.093 | 0.098 | 0.116 | 0.099 | 0.097 | 0.067 | 0.064 |

**Table 15 Detection of binding activity of lead antibody to mouse IL-5 by ELISA**

| OD₄₅₀ₙₘ | Clone number | | | | | | | | | |
|---|---|---|---|---|---|---|---|---|---|---|
| Ab conc. (nM) | 69F2F3 | | 102A2E11B6 | | 100F4E11B3 | | 154F10G8 | | hIgG control | |
| 13.3333 | 2.659 | 2.631 | 1.77 | 1.866 | 0.611 | 0.566 | 4.276 | 4.173 | 0.05 | 0.049 |
| 2.6667 | 2.074 | 2.124 | 1.304 | 1.336 | 0.294 | 0.368 | 4.152 | 4.196 | 0.06 | 0.058 |
| 0.5333 | 1.161 | 1.129 | 0.99 | 0.77 | 0.156 | 0.184 | 3.059 | 3.157 | 0.047 | 0.047 |
| 0.1067 | 0.535 | 0.601 | 0.41 | 0.452 | 0.091 | 0.094 | 1.346 | 1.318 | 0.047 | 0.052 |
| 0.0213 | 0.178 | 0.193 | 0.147 | 0.158 | 0.062 | 0.057 | 0.419 | 0.444 | 0.046 | 0.046 |
| 0.0043 | 0.083 | 0.076 | 0.069 | 0.069 | 0.051 | 0.048 | 0.166 | 0.158 | 0.044 | 0.045 |
| 0.0009 | 0.06 | 0.054 | 0.052 | 0.053 | 0.049 | 0.048 | 0.117 | 0.109 | 0.045 | 0.047 |
| 0.0002 | 0.079 | 0.048 | 0.046 | 0.046 | 0.047 | 0.049 | 0.097 | 0.106 | 0.045 | 0.046 |

Tables 13-15 show that the chimeric antibodies bind to recombinant human IL-5 protein and recombinant monkey IL-5 at ELISA level. Some chimeric antibodies bind to mouse IL-5 at ELISA level. Wherein, the hIgG control is human IgG, and the data in the table is OD₄₅₀ₙₘ values.

### 7.2 Biological function analysis

### 7.2.1 Receptor ligand binding blocking assay

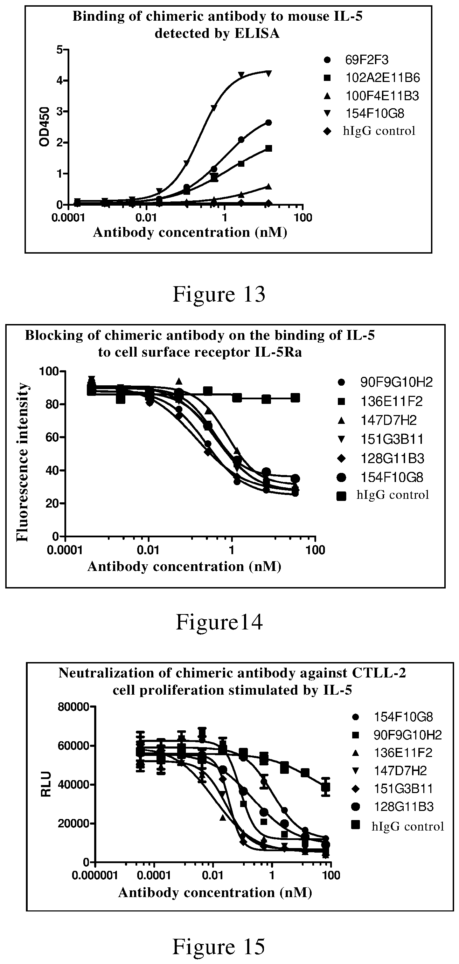

In this experiment, flow cytometry was used to detect the blocking of IL-5 chimeric antibody on the binding of biotinylated IL-5 to the receptor IL-5Ra heterodimer on the surface of CHOK1 cell line overexpressing full-length human IL-5Ra/CSF2RB. The detailed steps were the same as those in 3.2.2. Part of the results are shown in Figure 14 and Table 16.

**Table 16 Blocking of lead antibody on the binding of IL-5 to cell surface receptor IL-5Ra detected by FACS**

| Fluorescence intensity | Clone number | | | | | | |
|---|---|---|---|---|---|---|---|
| Antibody concentration (nM) | 90F9G10H2 | 136E11F2 | 147D7H2 | 151G3B11 | 128G11B3 | 154F10G8 | hIgG control |
| 33.3333 | 26 | 28 | 31 | 28 | 28 | 35 | 84 |
| 6.6667 | 28 | 32 | 36 | 33 | 31 | 39 | 83 |
| 1.3333 | 33 | 44 | 52 | 41 | 36 | 46 | 84 |
| 0.2667 | 56 | 66 | 76 | 68 | 51 | 67 | 88 |
| 0.0533 | 77 | 83 | 94 | 82 | 73 | 87 | 86 |
| 0.0107 | 81 | 85 | 86 | 85 | 81 | 88 | 85 |
| 0.0021 | 84 | 88 | 88 | 90 | 88 | 88 | 83 |
| 0.0004 | 94 | 88 | 94 | 95 | 93 | 91 | 88 |

Table 16 shows that the binding of human-mouse chimeric antibody to human IL-5 can block the binding of human IL-5 to the cell surface receptor IL-5Ra, wherein the hIgG control is human IgG, and the data in the table is average fluorescence intensity.

### 7.2.2 Neutralization experiment of chimeric antibody against CTLL-2 proliferation stimulated by IL-5

The neutralization experiment of chimeric antibody against CTLL-2 proliferation stimulated by IL-5 was that IL-5 chimeric antibody blocked the binding of IL-5 to its receptor and neutralized CTLL-2 cell line overexpressing full-length human IL-5Ra/CSF2RB stimulated by IL-5. See 4.2. 3 for detailed process. Part of the results are shown in Figure 15 and Table 17.

**Table 17 Neutralization of chimeric antibody against CTLL-2 cell proliferation stimulated by IL-5**

| RLU | Clone number | | | | | | | | | | | | | |
|---|---|---|---|---|---|---|---|---|---|---|---|---|---|---|
| Ab conc. (nM) | 154F10G8 | | 90F9G10H2 | | 136E11F2 | | 147D7H2 | | 151G3B11 | | 128G11B3 | | hIgG control | |
| 66.66666 | 12178.05 | 12440.13 | 5687.862 | 6461.691 | 3685.525 | 3956.583 | 5226.642 | 5239.829 | 3689.886 | 4029.338 | 8371.677 | 9777.978 | 43168.49 | 34361.45 |
| 13.33333 | 16166.05 | 16969.77 | 9500.172 | 10072.89 | 5788.416 | 5154.488 | 7305.869 | 6686.057 | 5748.64 | 5814.767 | 12158.55 | 14164.4 | 45781.71 | 46324.49 |
| 2.666667 | 27981.56 | 25408.79 | 14694.01 | 14221.84 | 8088.043 | 7445.372 | 8620.222 | 8163.056 | 6581.84 | 7939.647 | 18771.25 | 20944.62 | 51170.11 | 46188.8 |
| 0.5333334 | 44890.18 | 38172.15 | 21278.11 | 20381.87 | 11952.82 | 12083.97 | 9569.721 | 10444.49 | 8367.268 | 8248.239 | 25855.65 | 25595.55 | 56199.58 | 50465.38 |
| 0.1066667 | 54779.38 | 51966.38 | 30249.28 | 29742.14 | 16044.93 | 14396.72 | 12717.13 | 13187.49 | 10880.09 | 10289.36 | 37590.99 | 35519.01 | 58199.99 | 53498.81 |
| 0.02133333 | 62087.08 | 59317.76 | 57962.86 | 63952.38 | 23000.65 | 22987.53 | 33751.18 | 36441.43 | 41651.19 | 43934.77 | 46328.57 | 48867.84 | 59780.18 | 55928.2 |
| 0.004266667 | 64354.09 | 61348.89 | 64704.35 | 68879.53 | 44348.71 | 45756.46 | 41355.96 | 48226.65 | 51217.55 | 57980.14 | 51759.8 | 58174.11 | 57862.94 | 48241.73 |
| 0.000853333 | 59837.55 | 56731.89 | 59982.68 | 67200.71 | 50233.31 | 52646.61 | 50780.63 | 50723.48 | 54329.93 | 62961.71 | 50075.77 | 60554.68 | 58589.57 | 53792.09 |
| 0.000170667 | 59946.75 | 54888.58 | 58417.54 | 57975.97 | 45953.2 | 52336.2 | 50661.94 | 51743.31 | 47893.57 | 63164.5 | 45587.95 | 59602.46 | 57433.97 | 51016.91 |
| 3.41333E-05 | 60728.63 | 50197.32 | 55964.89 | 61915.06 | 58544.32 | 66868.45 | 56055.62 | 52165.31 | 46817.91 | 55030.88 | 48876.66 | 64517.9 | 58602.7 | 55004.59 |

Table 17 shows that by binding to human IL-5, the chimeric antibody can neutralizethe proliferation of CTLL-2 cells induced by IL-5 stimulation. The data in Table 17 are the relative light unit values of ATP measured in cell culture plate, wherein the hIgG control is human IgG.

### 7.2.3 Neutralization experiment of chimeric antibody against TF1 proliferation stimulated by IL-5

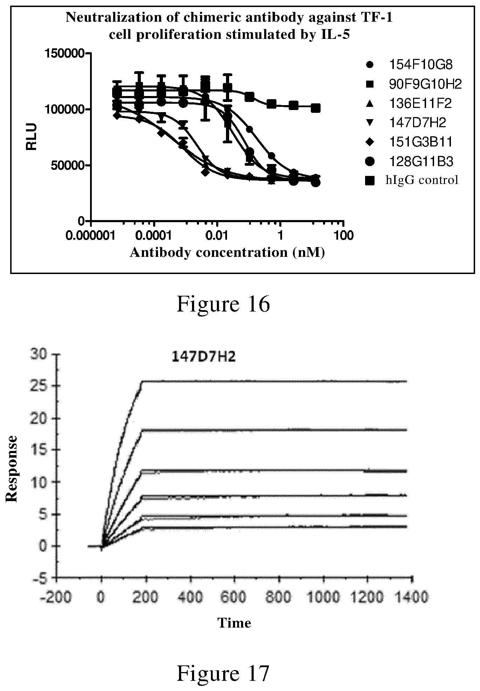

The neutralization experiment of chimeric antibody against TF1 proliferation stimulated by IL-5 was that IL-5 chimeric antibody bound to IL-5, blocked the binding of IL-5 to its receptor on the surface of TF-1 cells, and then neutralized IL-5-induced TF-1 cell proliferation. See 3.2.4 for detailed process. Part of the results are shown in Figure 16 and Table 18.

**Table 18 Neutralization of chimeric antibody against TF-1 cell proliferation stimulated by IL-5**

| | | | | | | | | | | | | | | |
|---|---|---|---|---|---|---|---|---|---|---|---|---|---|---|
| RLU | Clone number | | | | | | | | | | | | | |

| Ab conc. (nM) | 154F10G8 | | 90F9G10H2 | | 136E11F2 | | 147D7H2 | | 151G3B11 | | 128G11B3 | | hIgG control | |
|---|---|---|---|---|---|---|---|---|---|---|---|---|---|---|
| 13.33333 | 38491.96 | 39962.99 | 40225.86 | 33989.01 | 37675.66 | 36448.29 | 35692.59 | 39025.3 | 36181.86 | 33488.43 | 34960.05 | 34469.51 | 100840.8 | 101667.4 |
| 2.66667 | 45161.54 | 42628.1 | 44326.18 | 35820.97 | 35470.95 | 38489.36 | 34766.34 | 41236.5 | 38398.37 | 36772.32 | 36390.79 | 35595.93 | 101199.6 | 105936.7 |
| 0.53333 | 60398.51 | 56811.74 | 50222.09 | 39957.66 | 35120.92 | 37180.17 | 36441.77 | 40124.09 | 36408.96 | 36790.49 | 43485.45 | 48045.66 | 103434.2 | 106463.5 |
| 0.10667 | 81392.48 | 82781.78 | 62023.01 | 50958.51 | 40430.42 | 39562.17 | 38975.35 | 38662.06 | 41595.96 | 38802.61 | 59673.27 | 63897.36 | 108035 | 114497.9 |
| 0.02133 | 99081.16 | 101206 | 103044.4 | 70696.41 | 45535.36 | 45430.81 | 45259.36 | 43919.92 | 38570.97 | 44112.25 | 94905.84 | 93293.42 | 108062.2 | 130830.1 |
| 0.00427 | 109287.6 | 114295.4 | 128887.3 | 90493.41 | 50235.73 | 51954.05 | 56687.71 | 54812.5 | 41546 | 45988.11 | 108386.6 | 105865.8 | 116092.1 | 126837.9 |
| 0.00085 | 108878.9 | 108302.3 | 129887.4 | 107172 | 67978.02 | 67109.77 | 89215.6 | 82945.22 | 66250.13 | 74216.87 | 105370.7 | 106115.6 | 114520.6 | 117368.3 |
| 0.00017 | 110586.1 | 111330.7 | 129973.8 | 113108.8 | 81724.55 | 80679.02 | 96425.86 | 88816.04 | 76565.1 | 80444 | 104825.6 | 109167.8 | 112204.3 | 120642.9 |
| 0.00003 | 111308 | 108842.6 | 132674 | 110263.1 | 92175.36 | 95857.47 | 99962.88 | 95000.16 | 89809.68 | 92616.65 | 107437.3 | 101573.5 | 112208.9 | 116055.7 |
| 0.00001 | 110413.5 | 113028.7 | 125096.1 | 111663.3 | 100485.1 | 107631.1 | 99499.76 | 100598.5 | 95537.18 | 93897.51 | 107092.1 | 100533.4 | 113362.5 | 117922.4 |

Table 18 shows that by binding to human IL-5, the chimeric antibody can neutralize proliferation of TF-1 cells induced by IL-5 stimulation. The data in Table 18 are the relative light unit values of ATP measured in cell culture plate, wherein the hIgG control is human IgG.

### EXAMPLE 8 Chimeric antibody affinity test

The human antigen IL-5 was immobilized on the surface of CM5 chip to 150-200 RU and the process was as follows: a freshly prepared mixture of 50 mM NHS and 200 mM EDC (1: 1) was used to activate for 200 sec, the antigen IL-5 was diluted to lug/ml with 10 mM NaOAc (pH 5.0), and captured on the chip at a flow rate of 10ul/min for about 1min. The remaining activated sites were blocked with 1M ethanolamine. Then the antibody to be tested was diluted to different concentrations (0, 3.125, 6.25, 12.5, 25, 50nM), and then flowed through the chip surface at a flow rate of 30ul/min, respectively. The binding time was 180s and the dissociation time was 1200s. At the end of each cycle, the chip surface was regenerated with 10mM Glycine at pH 1.5. The kinetic rate constant needed to be subtracted the blank control, and the data was fitted with the global fit analysis method 1:1 combination model. The dissociation equilibrium rate constant (KD) was calculated according to the following formula: KD=kd/ka. Part of the results are shown in Figure 17 and Table 19.

**Table 19 Detection results of affinity between chimeric antibodies and human IL-5**

| Antibody | Ka (1/Ms) | Kd (1/s) | KD (M) |
|---|---|---|---|
| 69F2F3 | 8.19E+04 | <1E-05 | <1.2E-10 |
| 102A2E11B6 | 1.24E+05 | 2.73E-05 | 2.21E-10 |
| 100F4E11B3 | 3.11E+05 | 1.16E-04 | 3.71E-10 |
| 144D3C4A5 | 2.69E+05 | <1E-05 | <3.7E-11 |
| 90F9G10H2 | 9.44E+04 | <1E-05 | <1.06E-10 |
| 136E11F2 | 1.05E+05 | <1E-05 | <9.5E-11 |
| 147D7H2 | 1.57E+05 | <1E-05 | <6.37E-11 |
| 151G3B11 | 1.57E+05 | <1E-05 | <6.37E-11 |
| 128G11B3 | 1.93E+05 | 3.76E-05 | 1.94E-10 |
| 154F10G8 | 2.79E+05 | 5.24E-05 | 1.88E-10 |

Table 19 shows that the chimeric antibody has high affinity with human IL-5. The data in Table 19 are ka, kd and KD values when the chimeric antibody binds to IL-5, and the hIgG control is human IgG.

### EXAMPLE 9 Antibody of the present invention can more effectively neutralize TF1 proliferation experiment induced by IL-5

Compared with the comparative antibody, the antibody in the present invention can more effectively neutralize the TF1 proliferation experiment induced by IL-5. The results are shown in Table 20

| Antibody | TF1 (IC50, nM) | | | | |
|---|---|---|---|---|---|
| Mepolizumab | 0.045 | 0.050 | 0.036 | 0.028 | 0.048 |
| Amgen IL-5 antibody | 0.046 | 0.026 | 0.031 | 0.016 | 0.040 |
| 102A2E11B6 | | 0.006 | 0.001 | | |
| 147D7H2 | | | | 0.002 | 0.002 |
| 151G3B11 | | | | 0.000 | 0.001 |

Table 20 shows that IC50 of the antibodies in the present invention (102A2E11B6, 147D7H2, and 151G3B11) in this experiment are at the level of 10E⁻¹² M, and the comparative antibodies (Mepolizumab, Amgen IL-5 antibody) are at the level of 10E⁻¹¹M.

### Discussion

The technical solution of the invention: the present invention uses traditional hybridoma technology to prepare monoclonal antibodies.

Therapeutic monoclonal antibodies can be developed by various technologies and approaches, including hybridoma technology, phage display technology, single lymphocyte gene cloning technology and so on. However, the preparation of monoclonal antibodies from wild-type or transgenic mice by hybridoma technology is still the mainstream of the preparation methods of therapeutic monoclonal antibodies. According to the latest advances in monoclonal antibody technology, the present invention adopts optimized hybridoma technology to prepare the required anti-IL-5 antibody.

The traditional hybridoma preparation technology was established by Kohler and Milstein 40 years ago (Kohler and Milstein 1975, Nature 256: 495), and has now been widely used in the preparation and production of many related monoclonal antibodies in scientific research, diagnosis, and treatment. Although the basic method is still in use today, there have been changes, improvements and innovations in many aspects, including the use of different strains of animals such as genetically modified animals, the introduction of electrofusion technology, and the application of highefficiency screening technology equipment such as ClonePix equipment, which make the application of tumor technology more diverse and efficient. Monoclonal antibodies prepared from conventional animals such as mice can be cloned by conventional molecular biology methods to clone the antibody heavy chain variable region and light chain variable region genes, and the variable region genes can be grafted to human antibody constant region genes to form human-mouse chimeric antibody to greatly reduce the immunogenicity of the human body. In addition, the CDR domains of the variable region of the mouse antibody can be grafted onto the framework of the human antibody, thereby reducing the composition of the mouse antibody to less than 5%, greatly increasing the safety of the antibody used in human body. Antibodies obtained through this approach are called humanized antibodies and are currently the main product in the antibody drug market.

### Appendix

Sequence information of the present invention (protein and gene (DNA) sequences of antibody products of the present invention):

**Table 21 Protein (amino acid) and gene (nucleotide) sequence numbers of IL-5 antibody**

| Clone number | Heavy chain protein | | | | | Light chain protein | | | | |
|---|---|---|---|---|---|---|---|---|---|---|
| | Variable region | | VH-CDR1 | VH-CDR2 | VH-CDR3 | Variable region | | VL-CDR1 | VL-CDR2 | VL-CDR3 |
| | Amino acid sequence | Nucleotide sequence | | | | Amino acid sequence | Nucleotide sequence | | | |
| 147D7H2 | 1 | 2 | 3 | 4 | 5 | 6 | 7 | 8 | 9 | 10 |
| 102A2E11B6 | 11 | 12 | 13 | 14 | 15 | 16 | 17 | 18 | 19 | 20 |
| 69F2F3 | 21 | 22 | 23 | 24 | 25 | 26 | 27 | 28 | 29 | 30 |
| 154F10G8 | 31 | 32 | 33 | 34 | 35 | 36 | 37 | 38 | 39 | 40 |
| 151G3B11 | 41 | 42 | 43 | 44 | 45 | 46 | 47 | 48 | 49 | 50 |

**Table 22 CDR region sequences and the amino acid sequence numbers (SEQ ID NO:) of IL-5 antibody are as follows:**

| Clone number | CDR | Amino acid sequence information | SEQ ID NO. |
|---|---|---|---|
| 147D7H2 | VH-CDR1 | DYFIH | 3 |
| | VH-CDR2 | RIDTEDGETKYAPKFQG | 4 |
| | VH-CDR3 | YGNYVAMDY | 5 |
| | VL-CDR1 | SASSSVSYMF | 8 |
| | VL-CDR2 | LTSKLAS | 9 |
| | VL-CDR3 | QQWNSNPYT | 10 |
| 102A2E11B6 | VH-CDR1 | SYGVH | 13 |
| | VH-CDR2 | VIWSGGSTDYNAVFMS | 14 |
| | VH-CDR3 | NRYVLDY | 15 |
| | VL-CDR1 | KSSQSLLTSGDQKNHLA | 18 |
| | VL-CDR2 | GASTRES | 19 |
| | VL-CDR3 | QNDHSFPLT | 20 |
| 69F2F3 | VH-CDR1 | NYGVH | 23 |
| | VH-CDR2 | VIWSGGSTDYNAAFKS | 24 |
| | VH-CDR3 | NKDFLDY | 25 |
| | VL-CDR1 | KSSQGLLNSGNQKNYLT | 28 |
| | VL-CDR2 | WASTRES | 29 |
| | VL-CDR3 | QNDYHFPLT | 30 |
| 154F10G8 | VH-CDR1 | SYWIT | 33 |
| | VH-CDR2 | DIYPGSGSTFYNEKFKS | 34 |
| | VH-CDR3 | ETTLGY | 35 |
| | VL-CDR1 | RS SQSIVHNNGNTYLE | 38 |
| | VL-CDR2 | KVSNRFS | 39 |
| | VL-CDR3 | FQGSHVPFT | 40 |
| 151G3B11 | VH-CDR1 | DYYMH | 43 |
| | VH-CDR2 | RIDPEDGETKYAPKFQG | 44 |
| | VH-CDR3 | YGNFPDY | 45 |
| | VL-CDR1 | SASSSVSYMY | 48 |
| | VL-CDR2 | LTSKLAS | 49 |
| | VL-CDR3 | QQWNSNPYT | 50 |

Note: VH-CDR1 is heavy chain variable region-CDR1, VH-CDR2 is heavy chain variable region-CDR2, and VH-CDR3 is heavy chain variable region-CDR3; wherein, VL-CDR1 is light chain variable region-CDR1, VL-CDR2 is light chain variable region-CDR2, and VL-CDR3 is light chain variable region-CDR3.

### Protein (amino acid) and gene (nucleotide) sequences of five monoclonal antibodies of the invention

Protein and gene sequences of heavy chain variable region of IL-5 antibody 147D7H2 
SEQ ID NO. 1 Amino acid sequence 118aa
EVQLQQSGAELVKPGASVKLSCTASGFNIKDYFIHWVKQRTEQGLEWIGRIDTEDGETKYAPKFQGKATITIDTSSNTAYLYFSSLTSEDTA
MYYCARYGNYVAMDYWGQGTSVTVSS
 
SEQ ID NO. 2 Gene sequence
GAGGTTCAGCTGCAGCAGTCTGGGGCAGAGCTTGTGAAACCAGGGGCCTCAGTCAAGTTGTCCTGCACAGCTTCTGGCTTCAACATTAAAGA
CTACTTTATACACTGGGTGAAGCAGAGGACTGAACAGGGCCTGGAGTGGATTGGAAGGATTGATACTGAGGATGGTGAAACTAAATATGCCC
CGAAATTCCAGGGCAAGGCCACTATAACAATAGACACATCCTCCAATACAGCCTACCTGTATTTCAGCAGCCTGACATCTGAGGACACTGCC
ATGTATTACTGTGCTAGATATGGTAATTACGTCGCTATGGACTACTGGGGTCAAGGAACCTCAGTCACCGTCTCCTCA
Protein and gene sequences of light chain variable region of IL-5 antibody 147D7H2 
SEQ ID NO. 6 Amino acid sequence 106aa
QIVLTQSPALMSASPGEKVTMTCSASSSVSYMFWYQQKPSSSPKPWIYLTSKLASGVPARFSGSGSGTSYSLTISSVEAEDAATYYCQQWNS
NPYTFGGGTKLEIK
 
SEQ ID NO. 7 Gene sequence
CAAATTGTTCTCACCCAGTCTCCAGCACTCATGTCTGCATCTCCAGGGGAGAAGGTCACCATGACCTGCAGTGCCAGCTCAAGTGTAA
GTTACATGTTCTGGTACCAGCAGAAGCCAAGTTCCTCCCCCAAACCCTGGATTTATCTCACATCCAAGCTGGCTTCTGGAGTCCCTGC
TCGCTTCAGTGGCAGTGGGTCTGGGACCTCTTACTCTCTCACAATCAGCAGCGTGGAGGCTGAAGATGCTGCCACTTATTACTGTCAG
CAGTGGAACAGTAACCCGTACACGTTCGGAGGGGGGACCAAGCTGGAAATAAAA
Protein and gene sequences of heavy chain variable region of IL-5 antibody 102A2E11B6 
SEQ ID NO. 11 Amino acid sequence 115aa
QVQLRQSGPGLVQPSQSLSITCTVSGFSLTSYGVHWVRQSPGKGLEWLGVIWSGGSTDYNAVFMSRLSISKDSSKSQVFFKMNSLQTDDTAI
YYCARNRYVLDYWGQGTSVTVSS
 
SEQ ID NO. 12 Gene sequence
CAGGTGCAGCTGAGGCAGTCAGGACCTGGCCTGGTGCAGCCCTCACAGAGCCTGTCCATCACCTGCACAGTCTCTGGTTTCTCATTAA
CTAGCTATGGTGTACACTGGGTTCGCCAGTCTCCAGGAAAGGGTCTGGAGTGGCTGGGAGTGATATGGAGTGGTGGAAGCACAGACTA
TAATGCAGTTTTCATGTCCAGACTGAGCATCAGCAAGGACAGTTCCAAGAGCCAAGTTTTCTTTAAAATGAACAGTCTGCAAACTGAT
GACACAGCCATATATTACTGTGCCAGAAACCGTTACGTTTTGGACTACTGGGGTCAAGGAACCTCAGTCACCGTCTCCTCA
Protein and gene sequences of light chain variable region of IL-5 antibody 102A2E11B6 
SEQ ID NO. 16  Amino acid sequence 113aa
DIVMTQSPSSLSVSAGERVTMRCKSSQSLLTSGDQKNHLAWYQQKPGQPPKLLIYGASTRESGVPDRFTGSGSGTDFTLTITSVQAEDLAVY
YCQNDHSFPLTFGAGTKLELK
 
SEQ ID NO. 17 Gene sequence
GACATTGTGATGACACAGTCGCCATCCTCCCTGAGTGTGTCAGCAGGAGAGAGGGTCACTATGAGGTGCAAGTCCAGTCAGAGTCTGT
TAACCAGTGGAGATCAAAAGAACCACTTGGCCTGGTACCAGCAGAAACCAGGGCAGCCTCCTAAACTGTTGATCTACGGGGCATCCAC
TAGGGAATCTGGGGTCCCTGATCGCTTCACAGGCAGTGGATCTGGAACCGATTTCACTCTTACCATCACCAGTGTGCAGGCTGAAGAC
CTGGCAGTTTATTACTGTCAGAATGATCATAGTTTTCCGCTCACGTTCGGTGCTGGGACCAAGCTGGAGCTGAAA
Protein and gene sequences of heavy chain variable region of IL-5 antibody 69F2F3 
SEQ ID NO. 21 Amino acid sequence 115aa
QVQLKQSGPGLVQPSQSLSITCTVSGFSLSNYGVHWVRQSPGKGLEWLGVIWSGGSTDYNAAFKSRLSITKDNSKSQVVFRMNSLQTDDTAT
YYCARNKDFLDYWGQGTKFTVSS
 
SEQ ID NO. 22 Gene sequence
CAGGTGCAGCTGAAGCAGTCAGGACCTGGCCTAGTGCAGCCCTCACAGAGCCTGTCCATCACCTGCACAGTCTCTGGTTTCTCATTAA
GTAACTATGGTGTACACTGGGTTCGCCAGTCTCCAGGAAAGGGTCTGGAGTGGCTGGGAGTGATATGGAGTGGTGGAAGCACAGACTA
TAATGCAGCTTTCAAATCCAGACTGAGCATCACCAAGGACAATTCCAAGAGCCAAGTTGTCTTTAGAATGAACAGTCTGCAAACTGAT
GACACAGCCACTTATTACTGTGCCAGAAATAAGGACTTCTTGGACTACTGGGGCCAAGGCACCAAGTTCACAGTCTCCTCA
Protein and gene sequences of light chain variable region of IL-5 antibody 69F2F3 
SEQ ID NO. 26 Amino acid sequence 113aa
DIVMTQSPSSLTVTAGEKVTMTCKSSQGLLNSGNQKNYLTWYQQKPGQPPKLLIYWASTRESGVPDRFTGSGSGTDFTLTISSVQAEDLAVY
YCQNDYHFPLTFGAGTKLELK
 
SEQ ID NO. 27 Gene sequence
GACATTGTGATGACACAGTCTCCATCCTCCCTGACTGTGACAGCAGGAGAGAAGGTCACTATGACCTGCAAGTCCAGTCAGGGTCTGT
TAAACAGTGGAAATCAAAAGAACTACTTGACCTGGTACCAGCAGAAACCAGGGCAGCCTCCTAAACTGTTGATCTACTGGGCATCCAC
CAGGGAATCTGGGGTCCCTGATCGCTTCACAGGCAGTGGATCTGGAACAGATTTCACTCTCACCATCAGCAGTGTGCAGGCTGAAGAC
CTGGCAGTTTATTACTGTCAGAATGATTATCATTTTCCGCTCACGTTCGGAGCTGGGACCAAGCTGGAACTGAAA
Protein and gene sequences of heavy chain variable region of IL-5 antibody 154F10G8 
SEQ ID NO. 31  Amino acid sequence 115aa
QVQLQQPGAELVKPGASVKLSCKASGYTFTSYWITWVKQRPGQGLEWIGDIYPGSGSTFYNEKFKSEATLTVDTSSSTAYMQLSSLTSEDSA
VFYCSTETTLGYWGQGTTLTVSS
 
SEQ ID NO. 32 Gene sequence
CAGGTCCAACTGCAGCAGCCTGGGGCTGAGCTTGTGAAGCCTGGGGCTTCAGTGAAGCTGTCCTGCAAGGCTTCTGGCTACACCTTCA
CCAGCTACTGGATAACCTGGGTGAAGCAGAGGCCTGGACAAGGCCTTGAGTGGATTGGAGATATTTATCCTGGTAGTGGTAGTACTTT
CTACAATGAGAAGTTCAAGAGCGAGGCCACACTGACTGTAGACACATCCTCCAGCACAGCCTACATGCAGCTCAGCAGCCTGACATCT
GAGGACTCTGCGGTCTTTTACTGTTCAACCGAGACTACCCTGGGCTACTGGGGCCAAGGCACCACTCTCACAGTCTCCTCA
Protein and gene sequences of light chain variable region of IL-5 antibody 154F10G8 
SEQ ID NO. 36  Amino acid sequence 112aa
DVLMTQTPLSLPVSLGDQASISCRSSQSIVHNNGNTYLEWYLQKPGQSPKLLIYKVSNRFSGVPDRFSGSGSGTDFTLKISRVEAEDLGVYY
CFQGSHVPFTFGSGTKLEIK
 
SEQ ID NO. 37 Gene sequence
GATGTTTTGATGACCCAAACTCCACTCTCCCTGCCTGTCAGTCTTGGAGATCAAGCCTCCATCTCTTGCAGATCTAGTCAGAGCATTG
TACATAATAATGGAAACACCTATTTAGAATGGTACCTGCAGAAACCAGGCCAGTCTCCAAAGCTCCTGATCTACAAAGTTTCCAACCG
ATTTTCTGGGGTCCCAGACAGGTTCAGTGGCAGTGGATCAGGGACAGATTTCACACTCAAGATCAGCAGAGTGGAGGCTGAGGATCTG
GGAGTTTATTACTGCTTTCAAGGTTCACATGTTCCATTCACGTTCGGCTCGGGGACAAAGTTGGAAATAAAA
Protein and gene sequences of heavy chain variable region of IL-5 antibody 151G3B11 
SEQ ID NO. 41  Amino acid sequence 116aa
EVHLQQSGAELVKPGASVKLSCTASGFNIKDYYMHWVKQRTEQGLEWIGRIDPEDGETKYAPKFQGQATITADTSSNTANLQLSSLTSEDTA
VYYCARYGNFPDYWGPGTTLTVSS
 
SEQ ID NO. 42 Gene sequence
GAGGTTCACCTGCAGCAGTCTGGGGCAGAGCTTGTGAAGCCAGGGGCCTCAGTCAAGTTGTCCTGCACAGCTTCTGGCTTCAACATTA
AAGACTACTATATGCACTGGGTGAAGCAGAGGACTGAACAGGGCCTGGAGTGGATTGGAAGGATTGATCCTGAGGATGGTGAAACTAA
ATATGCCCCGAAATTCCAGGGCCAGGCCACTATAACAGCGGACACATCCTCCAACACTGCCAACCTGCAGCTCAGCAGCCTGACATCT
GAGGACACTGCCGTCTATTACTGTGCGAGATATGGTAACTTCCCTGACTACTGGGGCCCAGGCACCACTCTCACAGTCTCCTCA
Protein and gene sequences of light chain variable region of IL-5 antibody 151G3B11

SEQ ID NO. 46 Amino acid sequence 106aa
QIVLTQSPALMSASPGEKVTMTCSASSSVSYMYWYQQKPRSSPKPWIYLTSKLASGVPARFSGSGSGTSYSLTISSMEAEDAATYYCQQWNS
NPYTFGGGTKLEIK

SEQ ID NO. 47 Gene sequence
CAAATTGTTCTCACCCAGTCTCCAGCACTCATGTCTGCATCTCCAGGGGAGAAGGTCACCATGACCTGCAGTGCCAGCTCAAGTGTAA
GTTACATGTACTGGTACCAGCAGAAGCCAAGATCCTCACCCAAACCCTGGATTTATCTCACATCCAAACTGGCTTCTGGAGTCCCTGC
TCGCTTCAGTGGCAGTGGGTCTGGGACCTCTTACTCTCTCACAATCAGCAGCATGGAGGCTGAAGATGCTGCCACTTATTACTGCCAG
CAGTGGAATAGTAACCCGTACACGTTCGGAGGGGGGACCAAGCTGGAAATAAAA

## Claims

1. A heavy chain variable region of an antibody, wherein the heavy chain variable region comprises complementary determining regions or CDRs selected from the group consisting of:
VH-CDR1 shown in SEQ ID NO: 10n+3,
VH-CDR2 shown in SEQ ID NO: 10n+4, and
VH-CDR3 shown in SEQ ID NO: 10n+5;
wherein each n is independently 0, 1, 2, 3, or 4;
wherein any one of the above amino acid sequences further comprises a derivative sequence which is obtained through optional addition, deletion, modification and/or substitution of at least one amino acid and is capable of retaining the binding affinity to IL-5.

2. A heavy chain of an antibody, wherein the heavy chain comprises the heavy chain variable region of claim 1.

3. A light chain variable region of an antibody, wherein the light chain variable region comprises complementary determining regions or CDRs selected from the group consisting of:
VL-CDR1 shown in SEQ ID NO: 10n+8,
VL-CDR2 shown in SEQ ID NO: 10n+9, and
VL-CDR3 shown in SEQ ID NO: 10n+10;
wherein each n is independently 0, 1, 2, 3, or 4;
wherein any one of the above amino acid sequences further comprises a derivative sequence which is obtained through optional addition, deletion, modification and/or substitution of at least one amino acid and is capable of retaining the binding affinity to IL-5.

4. A light chain of an antibody, wherein the light chain comprises the light chain variable region of claim 3.

5. An antibody, wherein the antibody comprises:
(1) the heavy chain variable region of claim 1; and/or
(2) the light chain variable region of claim 3;
or the antibody comprises: the heavy chain of claim 2; and/or the light chain of claim 4,
wherein any one of the above amino acid sequences further comprises a derivative sequence which is obtained through optional addition, deletion, modification and/or substitution of at least one amino acid and is capable of retaining the binding affinity to IL-5.

6. The antibody of claim 5, wherein the antibody comprises the heavy chain variable region of claim 1 and the light chain variable region of claim 3;
wherein, the heavy chain variable region and the light chain variable region comprise CDRs selected from the group consisting of:
| VH-CDR1 Sequence number | VH-CDR2 Sequence number | VH-CDR3 Sequence number | VL-CDR1 Sequence number | VL-CDR2 Sequence number | VL-CDR3 Sequence number |
|---|---|---|---|---|---|
| 3 | 4 | 5 | 8 | 9 | 10 |
| 13 | 14 | 15 | 18 | 19 | 20 |
| 23 | 24 | 25 | 28 | 29 | 30 |
| 33 | 34 | 35 | 38 | 39 | 40 |
| 43 | 44 | 45 | 48 | 49 | 50 |
wherein any one of the above amino acid sequences further comprises a derivative sequence which is obtained through optional addition, deletion, modification and/or substitution of at least one amino acid and is capable of retaining the binding affinity to IL-5.

7. The antibody of claim 5, wherein the heavy chain variable region of the antibody comprises the amino acid sequence shown in SEQ ID NO: 1, 11, 21, 31, or 41; and/or the light chain variable region of the antibody comprises the amino acid sequence shown in SEQ ID NO: 6, 16, 26, 36, or 46.

8. The antibody of claim 6, wherein the antibody is selected from the group consisting of:
| Antibody number | Clone | VH sequence number | VL sequence number |
|---|---|---|---|
| 1 | 147D7H2 | 1 | 6 |
| 2 | 102A2E11B6 | 11 | 16 |
| 3 | 69F2F3 | 21 | 26 |
| 4 | 154F10G8 | 31 | 36 |
| 5 | 151G3B11 | 41 | 46. |

9. A recombinant protein, wherein the recombinant protein comprises:
(i) the heavy chain variable region of claim 1, the heavy chain of claim 2, the light chain variable region of claim 3, the light chain of claim 4, or the antibody of any one of claims 5-8; and
(ii) an optional tag sequence that assists expression and/or purification.

10. A polynucleotide, wherein the polynucleotide encodes a polypeptide selected from group consisting of:
(1) the heavy chain variable region of claim 1, the heavy chain of claim 2, the light chain variable region of claim 3, the light chain of claim 4, or the antibody of any one of claims 5-8; and
(2) the recombinant protein of claim 9.

11. The polynucleotide of claim 10, wherein the polynucleotide encoding the heavy chain variable region is shown in SEQ ID NO: 2, 12, 22, 32, or 42; and/or, the polynucleotide encoding the light chain variable region is shown in SEQ ID NOs: 7, 17, 27, 37, or 47.

12. The polynucleotide of claim 11, wherein the polynucleotide encoding the heavy chain variable region and the polynucleotide encoding the light chain variable region are selected from the group consisting of:
| Clone | Polynucleotide encoding VH Sequence number | Polynucleotide encoding VL Sequence number |
|---|---|---|
| 147D7H2 | 2 | 7 |
| 102A2E11B6 | 12 | 17 |
| 69F2F3 | 22 | 27 |
| 154F10G8 | 32 | 37 |
| 151G3B11 | 42 | 47. |

13. A vector, wherein the vector comprises the polynucleotide of any one of claims 10-12.

14. A genetically engineered host cell, wherein the host cell contains the vector of claim 13 or the genome thereof is integrated with the polynucleotide of any one of claims 10-12.

15. An antibody conjugate, wherein the antibody conjugate comprises:
(a) an antibody moiety, which is selected from the group consisting of: the heavy chain variable region of claim 1, the heavy chain of claim 2, the light chain variable region of claim 3, the light chain of claim 4, the antibody of any one of claims 5-8, and a combination thereof; and
(b) a coupling moiety coupled to the antibody moiety, and the coupling moiety is selected from the group consisting of a detectable label, a drug, a toxin, a cytokine, a radionuclide, an enzyme, and a combination thereof.

16. An immune cell, which expresses or is exposed outside the cell membrane with the antibody of any one of claims 5-8.

17. A pharmaceutical composition, wherein the pharmaceutical composition comprises:
(i) an active ingredient, wherein the active ingredient is selected from the group consisting of: the heavy chain variable region of claim 1, the heavy chain of claim 2, the light chain variable region of claim 3, the light chain of claim 4, and the antibody of any one of claims 5-8, the recombinant protein of claim 9, the antibody conjugate of claim 15, the immune cell of claim 16, and a combination thereof; and
(ii) a pharmaceutically acceptable carrier.
